# EUROPÄISCHE PATENTSCHRIFT

(11) **EP 3 873 316 B1**
(45) Veröffentlichungstag und Bekanntmachung des Hinweises auf die Patenterteilung: **04.10.2023**
(21) Anmeldenummer: 19797361.3
(22) Anmeldetag: 25.10.2019
(51) Int. Cl.: A61B 1/00, A61B 1/005

(54) **VERFAHREN ZUR HERSTELLUNG EINES EINFÜHRSCHLAUCHES EINES ENDOSKOPS UND ENDOSKOP MIT EINEM EINFÜHRSCHLAUCH**
METHOD FOR PRODUCING AN INSERTION TUBE OF AN ENDOSCOPE AND ENDOSCOPE HAVING AN INSERTION TUBE
PROCÉDÉ POUR LA FABRICATION D'UNE GAINE D'INTRODUCTION D'UN ENDOSCOPE ET ENDOSCOPE PRÉSENTANT UNE GAINE D'INTRODUCTION

(30) Priorität: 31.10.2018 DE 102018127227
(43) Veröffentlichungstag der Anmeldung: 08.09.2021
(73) Patentinhaber: HOYA CORPORATION, Shinjuku-ku Tokyo 160-8347 (JP)
(72) Erfinder: DO, Anh Minh, 86316 Friedberg (DE)
(74) Vertreter: TBK
(86) Internationale Anmeldenummer: PCT/IB2019/001086
(87) Internationale Veröffentlichungsnummer: WO 2020/089684

(56) Entgegenhaltungen:
- EP-A1- 2 777 476
- EP-A1- 3 135 182
- EP-A2- 2 742 879
- WO-A1-2012/073072

## Beschreibung

Die vorliegende Erfindung bezieht sich auf ein Verfahren zur Herstellung eines Einführschlauches eines Endoskops und auf ein Endoskop mit einem Einführschlauch.

Ein Endoskop ist ein Gerät, mit dem das Innere von lebenden Organismen, aber auch technische Hohlräume untersucht werden können. Ein wichtiges Teil eines Endoskops ist der flexible Einführschlauch. Die Anforderungen an einen Einführschlauch sind hoch und vielseitig. Einerseits muss er flexibel sein, um in den menschlichen Körper eingeführt werden zu können. Andererseits muss der Einführschlauch bestimmte Steifigkeit besitzen. Bei der Untersuchung muss der Arzt den Einführschlauch anhand des Kontrollkörpers schieben und drehen können. Dabei muss der Einführschlauch so steif sein, dass er nicht geknickt bzw. verdreht wird. Herkömmliche Einführschläuche bedingen daher eine sehr komplexe Aufbauweise und hohe Herstellungskosten, um die genannten Anforderungen zu erfüllen.

Um alle Anforderungen zu erfüllen, muss der Einführschlauch verschiedene Eigenschaften besitzen. Drei der wichtigsten Eigenschaften eines Einführschlauches sind die Biegeflexibilität, der Torsionswiderstand und die Formstabilität. Einerseits muss er biegbar sein, um in den zu untersuchenden (z.B. menschlichen) Körper eingeführt werden zu können. Andererseits muss der Einführschlauch einen hohen Torsionswiderstand besitzen, um das Drehmoment, das vom Anwender anhand des Drehens eines Kontrollkörpers erzeugt wird, weiter an das distale Ende übertragen zu können. Des Weiteren darf sich der Einführschlauch nicht verformen, wenn er gebogen oder verdreht wird.

Die Anforderung, dass ein Einführschlauch die oben genannten Eigenschaften gleichzeitig besitzen muß, stellt in sich einen technischen Widerspruch dar. Ein Element ist normalerweise steif und formstabil, wenn es hohen Torsionswiderstand besitzt. Hat das Element aber hohe Biegeflexibilität, dann besitzt es keinen hohen Torsionswiderstand und ist nicht formstabil.

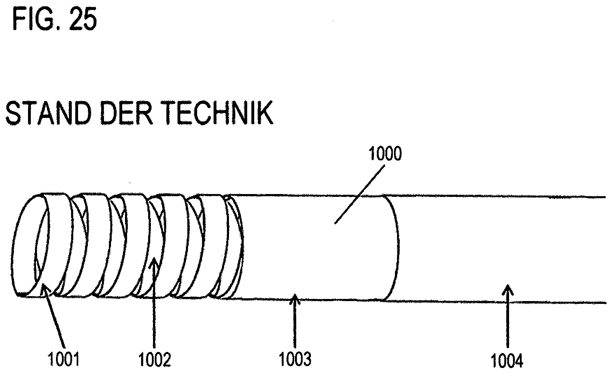

Um die oben genannte Anforderung zu erfüllen, versuchen die Entwickler seit geraumer Zeit den Basisbereich des Einführschlauchs mit mehreren Komponenten aufzubauen. Eine bekannte Aufbauweise eines Basisbereichs des Einführschlauchs ist in Fig. 25 zu erkennen.

Bei der bekannten Lösung aus Fig. 25 werden drei verschiedene Komponenten zusammengefügt, um die relevanten Eigenschaften des Basisbereichs eines Einführschlauchs 1000 zu erreichen, nämlich eine hohe Flexibilität, ein hoher Torsionswiderstand und eine hohe Formstabilität.

Ein Kunststoffüberzug 1004 wird erhitzt, bis das Material der Innenseite teilweise schmilzt und in Lücken eines Metallgeflechts 1003 eindringt. Diese Kombination verleiht dem Basisbereich eines Einführschlauchs 1000 einen hohen Torsionswiderstand und eine hohe Biegeflexibilität. Es fehlt hier allerdings noch die Formstabilität. Hier kommen zwei in Gegenrichtung angeordnete Metallblattspiralen 1001 und 1002 zum Einsatz. Diese Metallblattspiralen 1001 und 1002 sorgen dafür, dass der Einführschlauch formstabil wird. Die beschriebene Kombination verleiht nun dem Einführschlauch 1000 die drei genannten notwendigen Eigenschaften: nämlich eine hohe Flexibilität, ein hoher Torsionswiderstand und eine hohe Formstabilität.

Ein Nachteil bei dieser komplexen Aufbauweise liegt im wirtschaftlichen Aspekt. Drei Komponenten werden in einem aufwendigen Herstellungsprozess zusammengefügt. Sowohl die Materialien als auch der Herstellungsprozess verursachen hohe Herstellungskosten.

WO 2012/073072 A1 offenbart ein Endoskop mit den Merkmalen des Oberbegriffs von Anspruch 10.

Es ist die Aufgabe der vorliegenden Erfindung, ein Verfahren zur Herstellung eines Einführschlauches eines Endoskops und ein Endoskop mit einem Einführschlauch zu schaffen, die weniger komplex sind und durch die die Kosten gesenkt werden können.

In Hinblick auf das Verfahren ist die Aufgabe durch ein Verfahren mit den Merkmalen von Anspruch 1 gelöst. Ein Endoskop mit einem Einführschlauch ist in Anspruch 10 aufgezeigt. Vorteilhafte Weiterbildungen sind Gegenstand der abhängigen Ansprüche.

Die Erfindung ist auf ein Verfahren zur Herstellung eines Einführschlauches eines Endoskops aus einem Rohrelement gerichtet. Der Einführschlauch weist einen proximalen passiven flexiblen Abschnitt und einen distalen Abwinkelungsabschnitt auf. Im proximalen passiven flexiblen Abschnitt werden Schnitte vorgesehen, um ein Biegen des proximalen passiven flexiblen Abschnittes zu ermöglichen. Diese Schnitte werden im proximalen passiven flexiblen Abschnitt so ausgebildet, dass benachbarte Schnitte ungleich beabstandet sind.

Bei dem Einführschlauch werden Schnitte mit ungleichem Abstand erzeugt. Der Abstand von im Einführschlauch erzeugten Schnitten ist somit zueinander unterschiedlich. Die Schnitte können senkrecht zur Achse des Einführschlauches erzeugt werden.

In einem Beispiel können in Längsrichtung des Einführschlauches gesehen mehrere benachbart angeordnete Schnitte so erzeugt werden, dass ein Abstand zwischen einem ersten und einem zweiten benachbarten Schnitt ein vorbestimmter Abstand ist und ein Abstand zwischen dem zweiten und einem dritten benachbarten Schnitt kleiner oder größer als der vorbestimmte Abstand ist.

In einem anderen Beispiel können in Längsrichtung des Einführschlauches gesehen mehrere benachbart angeordnete Schnitte so erzeugt werden, dass ein Abstand zwischen einem ersten und einem zweiten benachbarten Schnitt ein vorbestimmter Abstand ist und ein Abstand zwischen dem zweiten und einem dritten benachbarten Schnitt genau so groß wie der vorbestimmte Abstand ist, aber ein Abstand zwischen dem dritten und einem vierten benachbarten Schnitt kleiner oder größer als der vorbestimmte Abstand ist.

Durch das ungleiche Beabstanden der Schnitte ergeben sich in Längsrichtung des Einführschlauches Bereiche mit großem Abstand zwischen den Schnitten und Bereiche mit kleinem Abstand zwischen den Schnitten. Der Bereich mit großem Abstand zwischen den Schnitten sorgt für eine hohe Biegestabilität und einen hohen Widerstand gegenüber Verdrehung. Der Bereich mit kleinem Abstand zwischen den Schnitten sorgt für eine hohe Biegbarkeit und eine hohe Flexibilität. Die genauen Maße für die Abstände können nach Bedarf gewählt werden.

Im proximalen passiven flexiblen Abschnitt werden Hauptschnitte vorgesehen, die in Längsrichtung des proximalen passiven flexiblen Abschnittes zueinander gleich beabstandet sind, und benachbart zu den Hauptschnitten werden Nebenschnitte im proximalen passiven flexiblen Abschnitt vorgesehen, die in Längsrichtung des proximalen passiven flexiblen Abschnittes zu den benachbarten Hauptschnitten an einer Seite der Nebenschnitte näher angeordnet sind als zu den benachbarten Hauptschnitten an der anderen Seite der Nebenschnitte.

Die Hauptschnitte können parallel zueinander geschnitten werden.

Die Hauptschnitte können entlang des Umfangs des proximalen passiven flexiblen Abschnittes in unterbrochener Weise so geschnitten werden, dass nicht geschnittene Stege zwischen auf einer Umfangslinie liegenden Hauptschnitt-abschnitten belassen bleiben.

Die Nebenschnitte werden jeweils benachbart zu einem Steg zwischen auf einer Umfangslinie liegenden Hauptschnitt-abschnitten geschnitten.

Ein Nebenschnitt kann in Längsrichtung des proximalen passiven flexiblen Abschnittes benachbart zu dem Steg an einer Seite des Steges jeweils geschnitten werden.

Alternativ können zwei Nebenschnitte in Längsrichtung des proximalen passiven flexiblen Abschnittes benachbart zu dem Steg an beiden Seiten des Steges jeweils geschnitten werden.

Die Hauptschnitte können breiter als die Nebenschnitte geschnitten werden.

Der gesamte Einführschlauch kann inklusive einem Anschlussbereich des proximalen passiven flexiblen Abschnittes an einem Kontrollkörper, dem proximalen passiven flexiblen Abschnitt, einem Übergangsbereich zwischen dem proximalen passiven flexiblen Abschnitt und dem Abwinkelungsabschnitt, und dem Abwinkelungsabschnitt aus einem einzigen Rohrelement hergestellt werden.

Der gesamte Einführschlauch kann durch Laser geschnitten werden.

In einem Beispiel des Verfahrens muss nur ein Rohrelement bereitgestellt werden. Ein Verbindungsvorgang zwischen dem proximalen passiven flexiblen Abschnitt und dem distalen Abwinkelungsabschnitt entfällt. Die Produktionskosten sind geringer als bei bisherigen Verfahren zur Herstellung eines Einführschlauches.

In diesem Verfahren kann der gesamte Einführschlauch inklusive dem Abwinkelungsabschnitt aus einem einzigen Rohrelement durch Laser geschnitten werden. Die Bearbeitung per Laser ermöglicht eine hochpräzise Gestaltung des gesamten Einführschlauches.

In diesem Verfahren können im Rohrelement einzelne Schnitte vorgenommen werden. Die Herstellung wird einfach und kostengünstig.

In diesem Verfahren weist der distale Abwinkelungsabschnitt nach innen gebogene Führungsvorsprünge auf, an denen ein Zugseil abgestützt ist; wobei die nach innen gebogenen Führungsvorsprünge aus der Umfangswand des distalen Abwinkelungsabschnittes geschnitten und dann nach innen gebogen werden. Somit werden auf einfache Weise Führungen für ein Zugseil an der Innenumfangsseite des Abwinkelungsabschnittes erzeugt.

In diesem Verfahren weist der Einführschlauch am Übergang vom proximalen passiven flexiblen Abschnitt und dem distalen Abwinkelungsabschnitt eine nach innen gebogene Lasche auf, an der eine Führungsfeder abgestützt ist; wobei die nach innen gebogene Lasche aus der Umfangswand des Einführschlauchs geschnitten und dann nach innen gebogen wird. Die Zahl der innen gebogenen Laschen, an der eine Führungsfeder abgestützt ist, entspricht der Anzahl an Führungsfedern und somit der Anzahl an Zugseilen. Somit werden auf einfache Weise Führungen für Führungsfedern an der Innenumfangsseite des Einführschlauches erzeugt.

In diesem Verfahren können in der Umfangswand des distalen Abwinkelungsabschnittes mehrere Gelenke durch Schneiden erzeugt werden. Auf einfache und kostengünstige Weise werden einzelne Gelenke erzeugt, die eigenständige Körper bilden und formschlüssig miteinander verbunden sind.

In diesem Verfahren das jeweilige durch Schneiden erzeugte Gelenk einen Kupplungsabschnitt, der mit einem benachbarten durch Schneiden erzeugten Gelenk so gekuppelt ist, dass eine axiale Bewegung aber keine radiale Bewegung der Gelenke zueinander blockiert ist, und einen Führungsabschnitt auf, der mit einem benachbarten durch Schneiden erzeugten Gelenk so in Eingriff steht, dass eine axiale Bewegung der Gelenke zueinander ermöglicht ist. Mit dem Kupplungsabschnitt sind benachbarte Gelenke miteinander gekuppelt und mit dem Führungsabschnitt sind benachbarte Gelenke zueinander axial bewegbar.

In diesem Verfahren wird der proximale passive flexible Abschnitt durch jeweilige seitliche Einschnitte, die senkrecht zur Längserstreckung des Rohrelementes erfolgen, erzeugt. Somit kann der proximale passive flexible Abschnitt schnell und einfach hergestellt werden.

In diesem Verfahren hat in Längserstreckung des Rohrelementes der proximale passive flexible Abschnitt zumindest zwei Unterabschnitte, die die jeweiligen seitlichen Einschnitte in zueinander unterschiedlichem Abstand in Längserstreckung des Rohrelementes haben. Somit können im proximalen passiven flexiblen Abschnitt mehrere separate Unterabschnitte mit zueinander unterschiedlicher Flexibilität und Biegbarkeit ausgebildet werden.

In diesem Verfahren kann das Rohrelement aus Edelstahl hergestellt werden. Die Schnitte können leicht erzeugt werden. Die Materialkosten sind niedrig.

In diesem Verfahren kann das Rohrelement aus Kunststoff hergestellt werden.

Ein beliebiger geeigneter Kunststoff mit ausreichender Festigkeit kann verwendet werden. Der Kunststoff muss lediglich die Biegefähigkeit des fertigen Einführschlauches erzeugen können.

In diesem Verfahren kann von einem proximal vom proximalen passiven flexiblen Abschnitt angeordneten Kontrollkörper ein Zugseil an der Innenumfangsseite des Rohrelementes angeordnet werden, das an einem am weitesten distal befindlichen Gelenk des distalen Abwinkelungsabschnittes durch einen ersten Schlitz in einer Wand des Rohrelementes zum Außenumfang des Rohrelementes geführt wird, um den Außenumfang des Rohrelementes herum zu einem zweiten Schlitz in der Wand des Rohrelementes zum Innenumfang des Rohrelementes geführt wird, wobei der zweite Schlitz um 180 Grad zu dem ersten Schlitz entgegengesetzt ist, und an der Innenumfangsseite des Rohrelementes wieder zum Kontrollkörper zurückgeführt wird. Auf diese Weise kann eine besonders kostengünstige Verankerung des Zugseils an der distalen Seite des Abwinkelungsabschnittes bewirkt werden.

Das erfindungsgemäße Endoskop ist in Anspruch 10 definiert. Der Einführschlauch des Endoskops weist einen proximalen passiven flexiblen Abschnitt und einen distalen Abwinkelungsabschnitt auf. Im proximalen passiven flexiblen Abschnitt sind Schnitte vorgesehen, um ein Biegen des proximalen passiven flexiblen Abschnittes zu ermöglichen. Benachbarte Schnitte sind im proximalen passiven flexiblen Abschnitt ungleich beabstandet.

Bei diesem Endoskop weist der proximale passive flexible Abschnitt Hauptschnitte auf, die in Längsrichtung des proximalen passiven flexiblen Abschnittes zueinander gleich beabstandet sind, und der proximale passive flexible Abschnitt weist benachbart zu den Hauptschnitten Nebenschnitte auf, die in Längsrichtung des proximalen passiven flexiblen Abschnittes zu den benachbarten Hauptschnitten an einer Seite der Nebenschnitte näher angeordnet sind als zu den benachbarten Hauptschnitten an der anderen Seite der Nebenschnitte.

Bei diesem Endoskop können die Hauptschnitte parallel zueinander sein.

Bei diesem Endoskop können die Hauptschnitte entlang des Umfangs des proximalen passiven flexiblen Abschnittes in unterbrochener Weise so verlaufen, dass nicht geschnittene Stege zwischen auf einer Umfangslinie liegenden Hauptschnitt-abschnitten belassen sind.

Bei diesem Endoskop sind die Nebenschnitte jeweils benachbart zu einem Steg zwischen auf einer Umfangslinie liegenden Hauptschnitt-abschnitten angeordnet.

Bei diesem Endoskop kann ein Nebenschnitt in Längsrichtung des proximalen passiven flexiblen Abschnittes benachbart zu dem Steg an einer Seite des Steges jeweils angeordnet sein.

Bei diesem Endoskop sind zwei Nebenschnitte in Längsrichtung des proximalen passiven flexiblen Abschnittes benachbart zu dem Steg an beiden Seiten des Steges jeweils angeordnet.

Bei diesem Endoskop können die Hauptschnitte breiter als die Nebenschnitte sein.

Bei diesem Endoskop kann der gesamte Einführschlauch inklusive einem Anschlussbereich des proximalen passiven flexiblen Abschnittes an einem Kontrollkörper, dem proximalen passiven flexiblen Abschnitt, einem Übergangsbereich zwischen dem proximalen passiven flexiblen Abschnitt und dem Abwinkelungsabschnitt, und dem Abwinkelungsabschnitt aus einem einzigen Rohrelement hergestellt sein.

Bei diesem Endoskop kann der gesamte Einführschlauch durch Laser geschnitten sein.

Ferner kann der gesamte Einführschlauch inklusive dem passiven flexiblen Abschnitt und dem Abwinkelungsabschnitt aus einem einzigen Rohrelement geformt sein.

Der distale Abwinkelungsabschnitt kann nach innen gebogene Führungsvorsprünge aufweisen, an denen ein Zugseil abgestützt ist.

Der Einführschlauch kann am Übergang vom proximalen passiven flexiblen Abschnitt und dem distalen Abwinkelungsabschnitt eine nach innen gebogene Lasche aufweisen, an der eine Führungsfeder abgestützt ist.

In der Umfangswand des distalen Abwinkelungsabschnittes können mehrere Gelenke ausgebildet sein.

Das jeweilige Gelenk kann einen Kupplungsabschnitt, der mit einem benachbarten Gelenk so gekuppelt ist, dass eine axiale Bewegung aber keine radiale Bewegung der Gelenke zueinander blockiert ist, und einen Führungsabschnitt aufweisen, der mit einem benachbarten Gelenk so in Eingriff steht, dass eine axiale Bewegung der Gelenke zueinander ermöglicht ist.

Das Rohrelement kann aus Edelstahl oder aus Kunststoff hergestellt sein.

Von einem proximal vom proximalen passiven flexiblen Abschnitt angeordneten Kontrollkörper kann ein Zugseil an der Innenumfangsseite des Rohrelementes angeordnet sein, das an einem am weitesten distal befindlichen Gelenk des distalen Abwinkelungsabschnittes durch einen ersten Schlitz in einer Wand des Rohrelementes zum Außenumfang des Rohrelementes geführt ist, um den Außenumfang des Rohrelementes herum zu einem zweiten Schlitz in der Wand des Rohrelementes zum Innenumfang des Rohrelementes geführt ist, wobei der zweite Schlitz um 180 Grad zu dem ersten Schlitz entgegengesetzt ist, und an der Innenumfangsseite des Rohrelementes wieder zum Kontrollkörper zurückgeführt ist.

Die vorstehend erläuterten Aspekte der vorliegenden Erfindung können geeignet kombiniert werden.

### Kurzbeschreibung der Zeichnung

Fig. 1 zeigt eine schematische Seitenansicht eines Endoskops.
Fig. 2 zeigt eine ausschnittartige schematische Ansicht eines Einführschlauches.
Fig. 3 zeigt eine ausschnittartige schematische Seitenansicht eines Teils eines proximalen passiven flexiblen Abschnittes des Einführschlauches eines ersten Ausführungsbeispiels.
Fig. 4 zeigt eine ausschnittartige perspektivische Ansicht des Teils des proximalen passiven flexiblen Abschnittes von Fig. 3.
Fig. 5 zeigt ein Detail des proximalen passiven flexiblen Abschnittes von Fig. 3 zur Erläuterung des Biegewiderstandes.
Fig. 6 zeigt einen Zusammenhang zwischen Verformung und Abstand zwischen Rohrschnitten beim Biegen in Hinblick auf den Biegewiderstand.
Fig. 7 zeigt ein Detail des proximalen passiven flexiblen Abschnittes von Fig. 3 zur weiteren Erläuterung des Biegewiderstandes.
Fig. 8 zeigt einen Zusammenhang zwischen Verformung und Abstand zwischen Rohrschnitten beim Biegen in Hinblick auf den Torsionswiderstand.
Fig. 9 zeigt ein Detail des proximalen passiven flexiblen Abschnittes von Fig. 3 zur Erläuterung des Torsionswiderstandes.
Fig. 10 zeigt eine ausschnittartige perspektivische Ansicht des Teils des proximalen passiven flexiblen Abschnittes des ersten Ausführungsbeispiels von Fig. 3 unter Torsionsbeanspruchung.
Fig. 11 zeigt eine ausschnittartige schematische Ansicht eines Übergangsbereiches zwischen dem distalen Abwinkelungsabschnitt und dem proximalen passiven flexiblen Abschnitt des Einführschlauches, wobei ein Führungsfederfixierabschnitt gezeigt ist.
Fig. 12 zeigt eine ausschnittartige perspektivische Ansicht des Führungsfederfixierabschnittes aus Fig. 11 von einer anderen Seite.
Fig. 13 zeigt eine ausschnittartige schematische Ansicht eines Teils des Abwinkelungsabschnittes des Einführschlauches.
Fig. 14 zeigt eine ausschnittartige schematische Ansicht des Teils des Abwinkelungsabschnittes des erfindungsgemäßen Einführschlauches, wobei eine Ansicht aus der Richtung eines Pfeiles I aus Fig. 13 gezeigt ist.
Fig. 15 zeigt eine ausschnittartige schematische Ansicht eines Teils des Abwinkelungsabschnittes des Einführschlauches, wobei eine Seilführung gezeigt ist.
Fig. 16 zeigt eine ausschnittartige perspektivische Ansicht der Seilführung aus Fig. 14.
Fig. 17 zeigt eine ausschnittartige schematische Seitenansicht des Abwinkelungsabschnittes des Einführschlauches.
Fig. 18 zeigt eine ausschnittartige schematische Draufsicht des Abwinkelungsabschnittes von Fig. 17.
Die Figuren 19 bis 21 zeigen jeweils eine ausschnittartige perspektivische Ansicht des distalen Endes des Abwinkelungsabschnittes.
Fig. 22 zeigt eine ausschnittartige perspektivische Ansicht der Zugseilverankerung am distalen Ende des Abwinkelungsabschnittes.
Fig. 23 zeigt eine Fig. 22 entsprechende Ansicht von einer anderen Seite.
Fig. 24 zeigt eine ausschnittartige schematische Darstellung des proximalen passiven flexiblen Abschnittes in einem zweiten Ausführungsbeispiel.
Fig. 25 zeigt ausschnittartige perspektivische Ansicht eines Einführschlauchs aus dem Stand der Technik.

Nachstehend sind Ausführungsbeispiele der Erfindung detailliert unter Bezugnahme auf die Zeichnungen anhand von Ausführungsbeispielen beschrieben.

### Erstes Ausführungsbeispiel

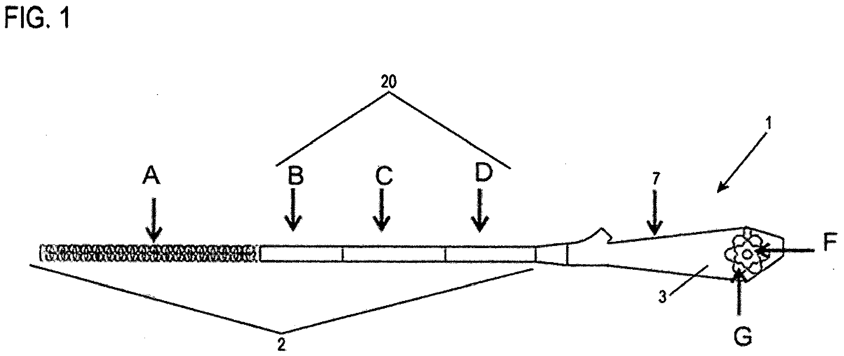

Nachstehend ist unter Bezugnahme auf die Figuren 1 bis 23 ein erstes Ausführungsbeispiel beschrieben.

Zunächst Fig. 1 zeigt eine schematische Seitenansicht eines Endoskops 1. Wie. aus Fig. 1 entnehmbar, hat ein solches Endoskop 1 einen Einführschlauch 2, der an der distalen Seite eines Kontrollkörpers 3 angeordnet ist. Der Kontrollkörper 3 dient als Bedieneinheit des Endoskops 1.

Der Einführschlauch 2 ist ein zylindrisches rohr- oder schlauchartiges Gebilde. Nachstehend ist der Einführschlauch 2 detaillierter in der Richtung, in der er bei einem Patienten eingeschoben wird, beschrieben. Der Einführschlauch 2 wird mit dem distalen Ende voran eingeschoben.

An der distalen Seite besitzt der Einführschlauch 2 einen distalen Abwinkelungsabschnitt A. Der Abwinkelungsabschnitt A kann mittels einem oder mehreren Steuerdrähten (Seilzug oder Seilzüge) relativ zum proximalen Teil des Einführschlauches 2 seitlich abgewinkelt werden. Der Steuerdraht oder Seilzug (nachfolgend nur als Steuerdraht bezeichnet) ist im Inneren des Einführschlauches 2 an einer Innenumfangsfläche des Einführschlauches 2 in Erstreckungsrichtung des Einführschlauches 2 geführt gelagert.

Das distale Ende des Steuerdrahts ist an der distalen Seite des Abwinkelungsabschnittes A verankert. Das proximale Ende des Steuerdrahts ist mit einem im Kontrollkörper 3 angeordneten Steuerelement verbunden. Dieses Steuerelement spannt den Steuerdraht, um eine erwünschte Abwinkelung des Abwinkelungsabschnittes A zu bewerkstelligen.

Proximal vom Abwinkelungsabschnitt A ist der Einführschlauch 2 als ein flexibles Schlauchelement gestaltet, das einen proximalen passiven flexiblen Abschnitt 20 ausbildet. Beim Einschieben des Einführschlauches 2 folgt der flexible Abschnitt 20 dem Abwinkelungsabschnitt A.

In Fig. 1 ist angedeutet, dass der flexible Abschnitt 20 entlang seiner Längsrichtung in Zonen mit unterschiedlicher Flexibilität gestaltet ist. Beispielsweise hat der flexible Abschnitt 20 unter Betrachtung in proximaler Richtung eine erste Zone B, eine zweite Zone C und eine dritte Zone D. Die erste Zone B bildet einen distalen Bereich, die zweite Zone C bildet einen mittleren Bereich und die dritte Zone D bildet einen proximalen Bereich.

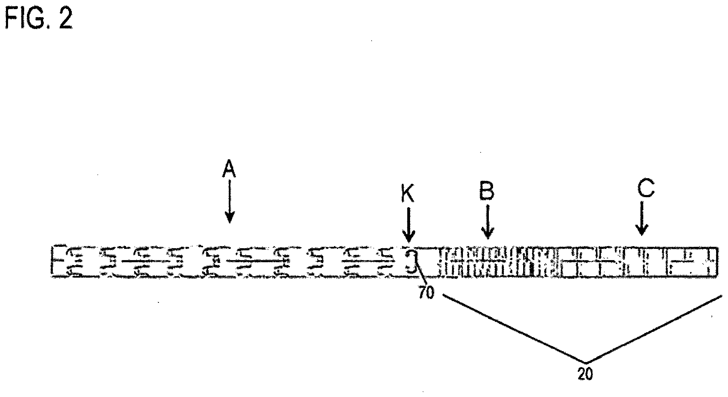

In der ausschnittartigen Darstellung von Fig. 2 ist die dritte Zone D nicht gezeigt.

Zur Vermeidung einer Knickbiegung zwischen dem Abwinkelungsabschnitt A und der ersten Zone B ist die erste Zone B vorzugsweise mit der höchsten Flexibilität unter den Zonen des flexiblen Abschnittes 20 versehen. Da die erste Zone B mit einer sehr hohen Flexibilität ausgestattet ist, ergibt sich kein abrupter Übergang der Flexibilität zwischen dem Abwinkelungsabschnitt A und der ersten Zone B.

Die zweite Zone C hat eine geringere Flexibilität als die erste Zone B. Die dritte Zone D hat eine wiederum geringere Flexibilität als die zweite Zone C.

Der Einführschlauch 2 ist aus einem Stück gebildet. Das heißt, am Übergang vom Abwinkelungsabschnitt A zum flexiblen Abschnitt 20 sind nicht zwei Elemente zusammengefügt. Somit sind der distale Abwinkelungsabschnitt A und der proximale passive flexiblen Abschnitt 20 mit den drei Zonen B, C und D aus einem einzigen Rohr oder Schlauch gebildet.

An der proximalen Seite ist der Einführschlauch 2 am distalen Ende des Kontrollkörpers 3 fixiert. Der Einführschlauch 2 kann am Kontrollkörper 3 z.B. durch einen Feststellring, einen Dichtungsring oder direkt fixiert sein. Der Einführschlauch 2 kann am Kontrollkörper 3 z.B. angeklebt oder angeschraubt sein. Der Kontrollkörper 3 hat ein erstes Steuerrad F als ein erstes Steuerelement zum Steuern eines Steuerdrahtes oder Seilzugs und ein zweites Steuerrad G als ein zweites Steuerelement zum Steuern eines Steuerdrahtes oder Seilzugs. Das erste Steuerrad F kann durch Ziehen eines Steuerdrahtes oder Seilzugs den Abwinkelungsabschnitt A in einer ersten Ebene abwinkeln (z.B. zum Betrachter hin und vom Betrachter weg in Fig. 1). Das zweite Steuerrad G kann durch Ziehen eines Steuerdrahtes oder Seilzugs den Abwinkelungsabschnitt A in einer zweiten Ebene, die senkrecht zur ersten Ebene ist, abwinkeln (z.B. in Fig. 1 nach oben und unten).

Der Abwinkelungsabschnitt A kann z.B. um 200 - 270 Grad abgewinkelt werden. Dies ist für die meisten Anwendungen ausreichend. In einer Spezialform kann der Abwinkelungsabschnitt A sogar um 300 Grad abgewinkelt werden.

Nachstehend ist der Einführschlauch 2 und seine Herstellung detaillierter beschrieben.

Der gesamte Einführschlauch 2 ist aus einem einzigen Rohrelement oder Schlauchelement (nachstehend ist dieses einfach als Rohrelement bezeichnet) gebildet. Das Rohrelement ist ein Rohr aus vorzugsweise relativ hartem Material. Besonders bevorzugt ist ein Rohr aus Edelstahl. Es kann aber auch ein Rohr aus hartem Kunststoff angewendet werden. Im Prinzip kann aber jedes für medizinische Zwecke anwendbare Material genutzt werden.

Durch eine Laserschneidmaschine werden im Rohrelement Schnitte vorgesehen, wie dies nachstehend detaillierter erläutert ist. Nach dem Vorsehen der Schnitte werden bestimmte Teilabschnitte des Rohrelementes gebogen, wie dies nachstehend detaillierter erläutert ist. Die Herstellung des Grundkörpers des gesamten Einführschlauches 2 macht keine weiteren Verfahrensschritte außer dem Vorsehen von Schnitten und dem Biegen erforderlich. Danach kann der Grundkörper des Einführschlauches 2 mit einem Steuerdraht versehen werden und einem Mantelelement ummantelt werden.

Nachstehend sind die einzelnen Abschnitte des Einführschlauches 2 genauer beschrieben.

### Flexibler Abschnitt 20

Der flexible Abschnitt 20 bildet den proximalen Teil des Einführschlauches 2. Der flexible Abschnitt 20 hat die drei Zonen B, C und D mit jeweils unterschiedlicher Flexibilität.

Fig. 1 zeigt den proximalen passiven flexiblen Abschnitt 20 der besseren Übersichtlichkeit so, als wenn die drei Zonen B, C und D entlang der Längsrichtung des Einführschlauches 2 zueinander gleich lang wären. Dies ist natürlich nicht der Fall. Die mittlere Zone C ist länger als der Übergangsbereich B und der Anschlussbereich D. Von den drei Zonen B, C und D ist die mittlere Zone C im proximalen passiven flexiblen Abschnitt 20 am längsten. Anders ausgedrückt wird der eigentliche proximale passive flexible Abschnitt 20 durch den Aufbau des mittleren Bereiches C gebildet. Die Biegeeigenschaften, die Elastizität und der Torsionswiderstand des proximalen passiven flexiblen Abschnittes 20 werden durch den Aufbau des mittleren Bereiches C verwirklicht.

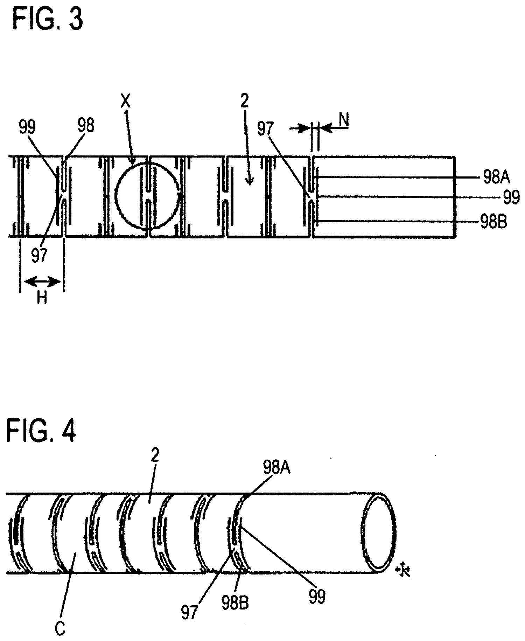

Nachstehend ist der Aufbau des mittleren Bereiches C und somit des eigentlichen proximalen passiven flexiblen Abschnittes 20 anhand der Figuren 3 - 10 genauer beschrieben.

Fig. 3 zeigt eine ausschnittartige schematische Seitenansicht eines Teils eines proximalen passiven flexiblen Abschnittes des Einführschlauches eines ersten Ausführungsbeispiels.

Fig. 4 zeigt eine ausschnittartige perspektivische Ansicht des Teils des proximalen passiven flexiblen Abschnittes von Fig. 3.

Die erfindungsgemäße Schnittkonstruktion des ersten Ausführungsbeispiels ist anhand der Figuren 3 und 4 zu erkennen.

Bei der Herstellung dieser Schnittkonstruktion wird ein Rohr 2 als Rohmaterial verwendet. Das Rohr 2 hat eine Achse und eine Längserstreckung. Das Rohr 2 besteht aus einem ausreichend harten Material. Beispielsweise kann Edelstahl verwendet werden. Kunststoff oder eine Nickel-Titan-Legierung wie z.B. Nitinol können ebenfalls genutzt werden. Das Rohr 2 bildet später den Einführschlauch.

Das Rohr 2 hat eine Form, die zunächst nicht flexibel ist. Das Rohr 2 hat einen hohen Torsionswiderstand und eine hohe Formstabilität.

In diesem Rohr 2 werden unter vorgegebenen Abständen H vorzugsweise per Laser Hauptschnitte 98 am Umfang in Umfangsrichtung erzeugt. Mit Umfangsrichtung ist eine Richtung gemeint, die rechtwinklig zur Achse des Rohres 2 läuft. Entlang des Rohres 2 ist der Abstand H jeweils gleich.

Die Hauptschnitte 98 durchdringen die Dicke des Mantels des Rohres 2. Die Hauptschnitte 98 erstrecken sich in Umfangsrichtung des Rohres 2 über annähernd eine halbe Umfangslänge. Somit werden pro Umfangslinie zwei in Umfangsrichtung aufeinander folgende Hauptschnitt-Abschnitte 98A, 98B erzeugt. Zwischen den jeweiligen Hauptschnitt-Abschnitten 98A, 98B befindet sich ein Steg 97, an dem das Material des Rohres 2 nicht geschnitten wird. In Längsrichtung des Rohres 2 gesehen ist über den Steg 97 der Bereich vor und hinter (proximal und distal von) dem jeweiligen Hauptschnitt 98 miteinander verbunden. An jeder Umfangslinie für den Hauptschnitt 98 sind somit zwei Stege 97 vorhanden. An jeder Umfangslinie für den Hauptschnitt 98 sind die beiden Stege 97 diametral entgegengesetzt angeordnet. In Umfangsrichtung gesehen entspricht eine Länge eines Hauptschnitt-Abschnittes 98A, 98B plus eine Länge des Steges 97 genau 180°. Die Länge des Hauptschnitt-Abschnittes 98A und des Hauptschnitt-Abschnittes 98B sind zueinander gleich.

Von Hauptschnitt 98 zu Hauptschnitt 98 sind entlang der Längsrichtung des Rohres 2 die Stege um 90° zueinander versetzt, wie dies in den Figuren 3 und 4 erkennbar ist.

In Längsrichtung des Rohres 2 werden proximal und distal von jedem Steg 97 Nebenschnitte 99 erzeugt. Die Nebenschnitte 99 verlaufen parallel zu den Hauptschnitt-Abschnitten 98A, 98B. Die Länge der Nebenschnitte 99 in Umfangsrichtung ist länger als die Länge des Steges 97 in Umfangsrichtung. Die Länge der Nebenschnitte 99 ist zueinander gleich.

In der Längsrichtung des Rohres 2 ist der Abstand N jedes Nebenschnittes 99 zu seinen benachbarten Hauptschnitt-Abschnitten 98A, 98B kleiner als der Abstand H der Hauptschnitte 98. Somit sind zu jedem Hauptschnitt 98 bestehend aus den beiden Hauptschnitt-Abschnitten 98A, 98B ein proximaler Nebenschnitt 99 und ein distaler Nebenschnitt 99 zugehörig.

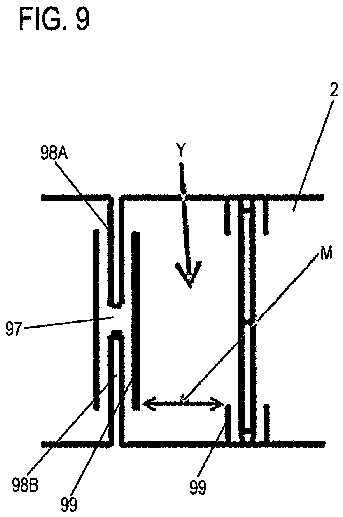

In der Längsrichtung des Rohres 2 ist der Abstand N jedes Nebenschnittes 99 zu seinen benachbarten Hauptschnitt-Abschnitten 98A, 98B ebenfalls kleiner als der Abstand M jedes Nebenschnittes 99 zu seinen benachbarten Nebenschnitt 99, der zum nächsten Hauptschnitt 98 zugehörig ist, siehe Fig. 9.

Durch die Hauptschnitte 98 und Nebenschnitte 99 ändert sich die Eigenschaft des Rohres 2. Das Rohr 2 wird flexibel. Die Flexibilität und andere Eigenschaften des Rohres 2 hängen stark von u. a. der Konstruktion der Schnitte 98, 99 ab. Genauer gesagt sind die Schnittbreite, Schnittlänge und die Abstände der RohrSchnitten u. a. (neben dem Material) entscheidende Faktoren, die sich auf die Eigenschaften des Rohres 2 auswirken.

In dem Bereich X befindet sich die Schnittkonstruktion, die für die Entstehung der hohen Flexibilität des Rohres 2 zuständig ist.

Nachstehend ist der Zusammenhang zwischen der Verformung und dem Abstand zwischen Rohrschnitten beim Biegen erläutert.

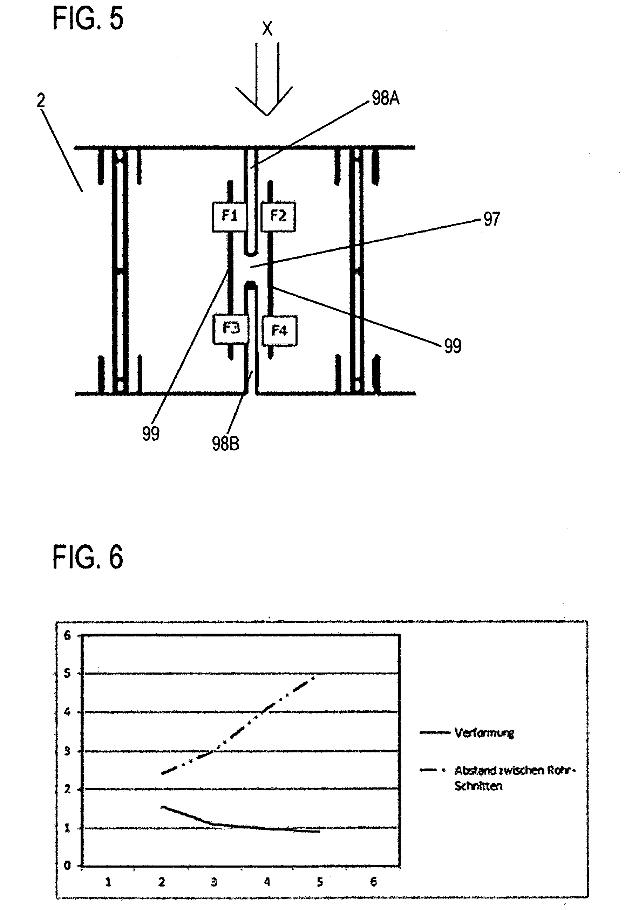

Ein Rohr hat in seiner ursprünglichen Form ohne Schnitte einen bestimmten Biegewiderstand. Sobald dieses Rohr geschnitten wird, sinkt der Biegewiderstand entsprechend der Form und Anzahl der im Rohr vorgesehenen Schnitte. Die graphische Darstellung in Fig. 6 zeigt den Zusammenhang zwischen der Verformung und dem Abstand zwischen Rohrschnitten, wenn das Rohr gebogen wird.

Fig. 6 zeigt die Ergebnisse einer Biegesimulation eines mit Schnitten versehenen Rohres. Die Verformung eines mit Schnitten versehenen Rohres bei einem Biegevorgang ist dargestellt.

Die Strichpunktlinie mit zwei Punkten gibt den Abstand eines Schnittes zu seinem benachbarten Schnitt an.

Die durchgezogene Linie gibt die Verformung des Rohres beim Biegen an.

Die Ordinate und die Abszisse Zeigen jeweils Längeneinheitsgrößen (z.B. mm).

Aus Fig. 6 ist folgendes erkennbar: Je größer der Abstand zwischen den Rohrschnitten ist, desto größer wird der Biegewiderstand (desto geringer wird die Verformung). Wird der Abstand zwischen den Rohrschnitten unendlich, erreicht das Rohr 2 seinen ursprünglich höchsten Biegewiderstand.

Da für einen Einführschlauch eines Endoskops ein niedriger Biegewiderstand (und damit eine hohe Flexibilität) benötigt wird, muss folglich ein Abstand zwischen den Rohr-Schnitten so klein wie möglich sein.

Die Konstruktion ist in dem Bereich X so ausgelegt, dass die Schnitte 89 und 99 dicht beieinander liegen (kleiner Abstand N) und vier federähnliche Abschnitte F1, F2, F3 und F4 entstehen. Wird das geschnittene Rohr 2 nun gebogen, werden die Abschnitte F1', F2, F3 und F4 auseinander gezogen und dadurch entsteht eine federähnliche Gegenkraft. Wird das Rohr 2 nach dem Biegen entlastet, wirkt die Gegenkraft auf das Rohr 2 so, dass es wieder seine gerade Form zurückerhält. Entlang der Längsrichtung des Rohres 2 ist diese Konstruktion des Bereiches X wiederholt um 90° versetzt und zwar auf der gesamten Länge des proximalen passiven flexiblen Abschnittes C des Rohrs 2. Dadurch ist das Rohr 2 in allen Richtungen gleichmäßig flexibel.

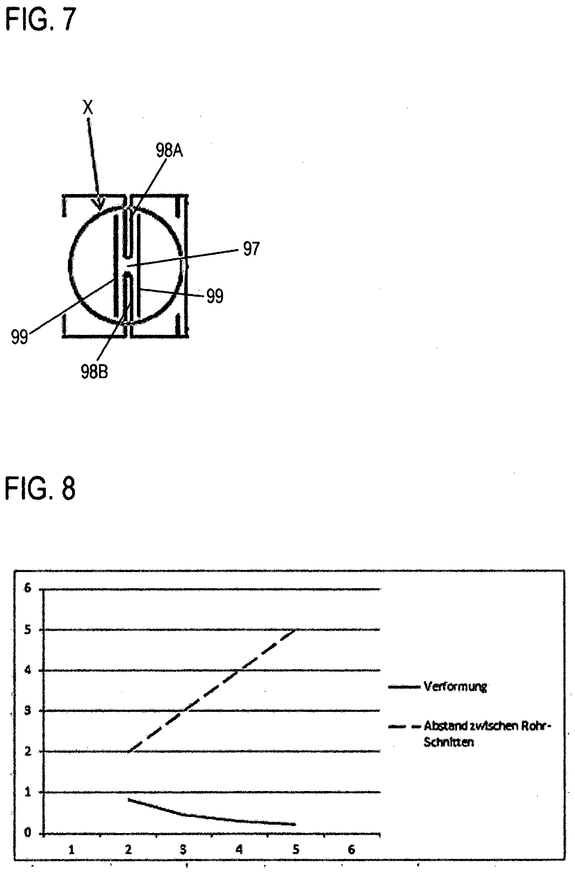

Fig. 7 zeigt den Bereich X als vergrößerten Ausschnitt. In der Konstruktion aus einem Hauptschnitt 98, der sich aus einem ersten Hauptschnitt-Abschnitt 98A und einem zweiten Hauptschnitt-Abschnitt 98B zusammensetzt, mit den zugehörigen Nebenschnitten 99 im Bereich X soll der Abstand N zwischen den Hauptschnitt-Abschnitten 98A, 98B und den zugehörigen Nebenschnitten 99 so klein wie möglich sein, um eine hohe Flexibilität zu erzeugen.

Nachstehend ist der Torsionswiderstand an einem Rohr erläutert.

Fig. 8 zeigt einen Zusammenhang zwischen der Verformung und dem Abstand zwischen Rohrschnitten beim Biegen in Hinblick auf den Torsionswiderstand. Anders ausgedrückt zeigt die graphische Darstellung von Fig. 8 den Zusammenhang zwischen der Verformung und dem Abstand zwischen Rohrschnitten, wenn das Rohr verdreht wird.

Fig. 8 zeigt die Ergebnisse einer Verdrehsimulation eines mit Schnitten versehenen Rohres. Die Verformung eines mit Schnitten versehenen Rohres bei einem Verdrehvorgang ist dargestellt.

Die gestrichelte Linie gibt den Abstand eines Schnittes zu seinem benachbarten Schnitt an.

Die durchgezogene Linie gibt die Verformung des Rohres bei Verdrehung an.

Die Ordinate und die Abszisse zeigen jeweils Längeneinheitsgrößen (z.B. mm).

Aus Fig. 8 ist folgendes erkennbar: Ein Rohr hat in seiner ursprünglichen Form ohne Schnitte einen bestimmten Torsionswiderstand. Sobald dieses Rohr geschnitten wird, sinkt der Torsionswiderstand entsprechend der Form und Anzahl der Schnitte. Je größer der Abstand zwischen Rohrschnitten ist, desto größer wird der Torsionswiderstand (und desto geringer wird die Verformung bei Drehung). Wird der Abstand zwischen Rohrschnitten unendlich, erreicht das Rohr seinen ursprünglich höchsten Torsionswiderstand.

Da für einen Einführschlauch eines Endoskops ein hoher Torsionswiderstand benötigt wird, soll folglich der Abstand zwischen Rohrschnitten so groß wie möglich sein.

Fig. 9 zeigt in einem Bereich Y in einem vergrößerten Ausschnitt den Abstand M jedes Nebenschnittes 99 zu seinen benachbarten Nebenschnitt 99, der zum nächsten Hauptschnitt 98 zugehörig ist.

Die Konstruktion im Bereich Y zeigt, dass der Abstand M zwischen benachbarten Nebenschnitten 99 so groß wie möglich sein soll, um einen hohen Torsionswiderstand zu erzeugen. Der genaue Abstand M zwischen benachbarten Nebenschnitten 99 kann nach individuellem Bedarf festgelegt werden.

Nachstehend ist das Erzielen der Formstabilität am Rohr 2 erläutert.

Ein hartes Rohr ist aus der Natur her formbeständig. Die Konstruktion des Bereiches Y ist so ausgelegt, dass das Rohr 2 die Formstabilität beibehält, nachdem an ihm eine Vielzahl an Schnitten 98, 99 vorgesehen worden sind.

### Breit

Die Nebenschnitte 99 sind hierbei so weit beabstandet angeordnet, dass der Bereich Y in Längsrichtung des Rohres 2 relativ lang ist. Anders ausgedrückt ergibt sich ein breiter Ringbereich im Bereich Y, der frei von Schnitten ist.

Der Bereich Y kann wie ein kurzes Rohr betrachtet werden und hat daher hohe Formstabilität. Wird das gesamte Rohr 2 gebogen, werden die Abschnitte F1, F2, F3 und F4 nachgeben, weil der Bereich Y eine Eigenstabilität aufweist.

Das Rohr 2 ist somit biegeflexibel und zugleich formstabil.

Nachstehend ist die Zusammenwirkung der Bereiche X und Y erläutert.

Die gesamte Konstruktion des proximalen passiven flexiblen Abschnittes C ist eine Kombination zwischen den Bereichen X und Y.

Jeder dieser Bereiche X und Y liefert dem Rohr 2 eine bestimmte Eigenschaft.

In dem Bereich X sind die Hauptschnitte 98 und Nebenschnittes 99 eng zueinander angeordnet, um eine hohe Flexibilität zu erreichen.

In dem Bereich Y sind die Nebenschnitte 99 dagegen weiter zueinander beabstandet, um einen hohen Torsionswiderstand zu erreichen.

Dadurch ergeben sich die folgenden Wechselwirkungen zwischen dem Bereich X und dem Bereich Y:
In dem Bereich Y sind die Nebenschnitte 99 weit voneinander beabstandet. Dieser Bereich Y ist somit sowohl beim Biegen als auch beim Verdrehen stabil. Beim Biegen bleibt der Bereich Y nahezu unverändert. Der Bereich X gibt dagegen nach und definiert die Flexibilität des gesamten Rohres 2. Die Auswirkung des Bereiches Y auf die Flexibilität des Rohres 2 ist unbedeutend.

In dem Bereich X sind die die Hauptschnitte 98 und Nebenschnittes 99 sehr eng zueinander angeordnet.

Die Hauptschnitte 98 und die Nebenschnitte 99 haben im Ausführungsbeispiel eine zueinander unterschiedliche Schnittbreite. Mit Schnittbreite ist die Breite des jeweiligen Schnittes in Längsrichtung des Rohres gemeint. Wenn die Hauptschnitte 98 und die Nebenschnittes 99 per Laser erzeugt werde, wird die Schnittbreite durch die Wahl des Durchmessers des emittierten Laserstrahlbündels eingestellt.

Die Schnittbreite der Nebenschnitte 99 soll so klein wie möglich gehalten werden. Mittels Laser kann z.B. eine Schnittbreite von weit unter 20 µm geschaffen werden. Beispielsweise können die Nebenschnitte 99 mit einer Schnittbreite von 20 µm erzeugt werden. Die Hauptschnitte 98 können beispielsweise mit einer Schnittbreite von 0,2 mm erzeugt werden. Diese Werte der Schnittbreite stellen lediglich Beispiele dar. Die jeweils geeigneten Schnittbreiten können durch Versuche ermittelt werden.

Vorzugsweise ist die Schnittbreite der Hauptschnitte 98 größer als die Schnittbreite der Nebenschnitte 99. Beispielsweise kann die Schnittbreite der Hauptschnitte 98 das zehnfache der Schnittbreite der Nebenschnitte 99 betragen. Auch dieser Wert ist lediglich ein Beispiel. Der jeweils geeignete Faktor kann nach Bedarf eingestellt werden. Die Erfindung ist nicht auf diese Werte beschränkt.

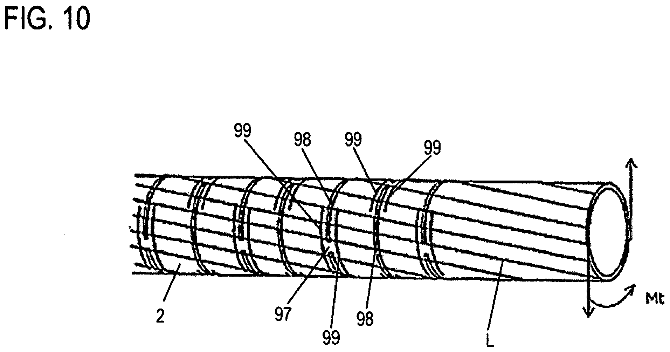

Bei einer Torsionsbeanspruchung wird das Rohr 2 mit einem Torsionsmoment Mt belastet, das um die Längsachse des Rohres 2 wirkt. Durch die Einwirkung des Torsionsmoments verformen sich gedachte parallel zur Längsachse laufende Längslinien L des Rohres 2 schraubenförmig, wie dies Fig. 10 zeigt. Da der Abstand N der Hauptschnitte 98 und Nebenschnitte 99 im Bereich X sehr klein ist, wird sich die Verformung des Bereichs X nur geringfügig von der des Bereichs Y unterscheiden. Der Torsionswiderstand des Bereichs Y definiert den Torsionswiderstand des gesamten Rohres 2. Die Auswirkung des Bereiches X auf den Torsionswiderstand des Rohres 2 ist unbedeutend.

Durch das wie vorstehend erläuterte Erzeugen von Schnitten mit zueinander unterschiedlichem Abstand kann im proximalen passiven flexiblen Abschnitt C des Rohrs 2 sowohl eine hohe Flexibilität als auch ein hoher Torsionswiderstand erreicht werden.

Somit ist der Endoskopschlauch 2 im proximalen passiven flexiblen Abschnitt C des flexiblen Abschnitts 20 lateral zu seiner Längsachse mit einer hohen Flexibilität und auch mit einem hohen Torsionswiderstand biegbar. Die einzelnen Zonen B, C und D im flexiblen Abschnitt 20 unterscheiden sich dadurch, dass die Abstände H der Schnitte 98in Längsrichtung und somit die Dichte der Schnitte 98 unterschiedlich gestaltet sind.

In der Zone B ist der Abstand H der Schnitte 98 am geringsten. Somit ist in der Zone B die Dichte der Schnitte 98 am höchsten.

In der Zone C ist der Abstand H der Schnitte 98 größer als in der Zone B. In der Zone D ist der Abstand H der Schnitte 98 größer als in der Zone C.

Somit ist die Flexibilität und die Biegbarkeit in der Zone B höher als in der Zone C. Ferner ist die Flexibilität und die Biegbarkeit in der Zone C höher als in der Zone D. Anders ausgedrückt nehmen die Flexibilität und die Biegbarkeit der jeweiligen Zonen am flexiblen Abschnitt 20 in proximaler Richtung ab.

Die Zone D ist an der proximalen Seite mit einem Bereich versehen, der nicht mit Schnitten versehen ist. Dieser Bereich bildet einen Übergang zum Kontrollkörper J.

Übergang vom Abwinkelungsabschnitt A zum flexiblen Abschnitt 20

Der Übergangsbereich vom Abwinkelungsabschnitt A zum flexiblen Abschnitt 20 ist in Fig. 2 als Bereich K angedeutet. In diesem Bereich K endet der Abwinkelungsabschnitt A. Anders ausgedrückt befindet sich distal vom Bereich K das erste d.h. am weitesten proximal befindliche Glied des Abwinkelungsabschnittes A.

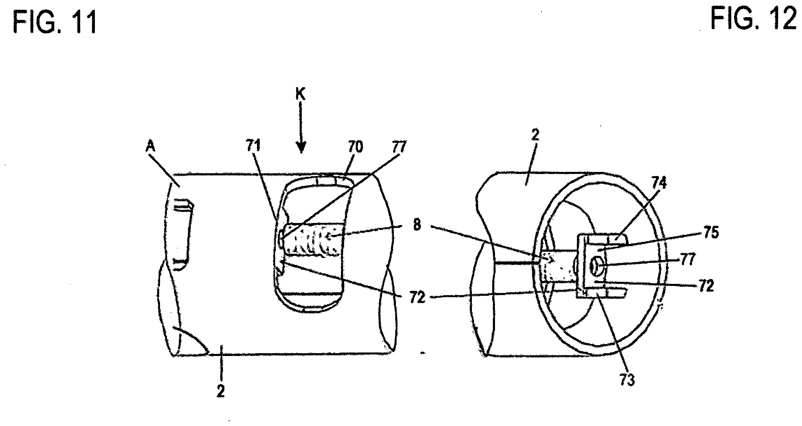

Wie in den Figuren 2, 11 und 12 gezeigt, ist in diesem Bereich K die Wandfläche des Rohrelements durch einen Schnitt 70 in der Form eines umgekehrten Buchstaben C geschnitten. Anders ausgedrückt, ist der Schnitt 70 im Rohrelement in der Form eines unvollständigen Kreises geschnitten. Der Kreis des Schnittes 70 ist an der distalen Seite nicht durchgeschnitten, wie aus Fig. 11 entnehmbar ist. Die nicht durchgeschnittene distale Seite des Schnittes 70 bildet ein Scharnier 71 für eine Lasche 72. Die Lasche 72 hat ein unteres Ohr 73, ein oberes Ohr 74 und ein Laschenmittelstück 75. An einer oberen Seite des Laschenmittelstücks 75 grenzt das untere Ohr 73 an. An einer unteren Seite des Laschenmittelstücks 75 grenzt das obere Ohr 74 an.

Die Lasche 72 wird wie folgt hergestellt. Der Ort des Schnittes 70 wird festgelegt. In der Mitte des Schnittes 70 wird ein Loch 77 geschnitten. Der Schnitt 70 wird per Laser wie in Fig. 2 gezeigt gebildet. Das Laschenmittelstück 75 wird von der Rückseite, d.h. von der Innenseite des Rohrelements durch einen Stempel abgestützt. Das untere Ohr 73 wird relativ zum Laschenmittelstück 75 um 90 Grad nach innen gebogen. Die Biegelinie des Ohres 73 relativ zum Laschenmittelstück 75 verläuft dabei parallel zur Achse des Rohrelements (in Figuren 2 und 4 in der nach links und nach rechts weisenden Richtung). Das obere Ohr 74 wird ebenfalls relativ zum Laschenmittelstück 75 um 90 Grad nach innen gebogen. Die Biegelinie des Ohres 74 relativ zum Laschenmittelstück 75 verläuft ebenfalls parallel zur Achse des Rohrelements. Danach wird das Laschenmittelstück 75 um 90 Grad nach innen gebogen. Die Biegelinie des Laschenmittelstücks 75 relativ zum Rohrelement verläuft in der senkrechten Schnittebene zur Achse des Rohrelements (in Figuren 2 und 11 in der nach oben und nach unten weisenden Richtung). Anders ausgedrückt wird das Laschenmittelstück 75 am Scharnier 71 um 90 Grad nach innen gebogen. Das Laschenmittelstück 75 wird insbesondere so weit nach innen gebogen, bis eine distale Seitenkante des unteren Ohres 73 und eine distale Seitenkante des oberen Ohres 74 am Innenumfang des Rohrelements anliegen, siehe Fig. 12).

Die Lasche 72 dient als Abstützung einer Führungsfeder 8. Insbesondere bildet die proximale Fläche des Laschenmittelstücks 75 eine Anschlagfläche für das distale Ende der Führungsfeder 8. Die beiden Ohren 73, 74 stützen das Laschenmittelstück 75 und nehmen von der Führungsfeder 8 wirkende Drückkräfte auf und leiten diese an die Innenumfangsfläche des Rohrelements weiter.

Das Laschenmittelstück 75 besitzt das zentrische Loch 77. Das Loch 77 hat einen größeren Durchmesser als ein Steuerdraht und einen kleineren Durchmesser als die Führungsfeder 8. Der Steuerdraht wird im flexiblen Abschnitt 20 in der Führungsfeder 8 geführt und durchläuft das Loch 70 und erstreckt sich weiter in den Abwinkelungsabschnitt A hinein.

Im Bereich K sind Laschen 72 in der Anzahl der verwendeten Steuerdrähte (im vorliegenden Ausführungsbeispiel: vier) vorgesehen. Die Laschen 72 sind in Umfangsrichtung des Rohrelements gleichmäßig verteilt.

### Abwinkelungsabschnitt A

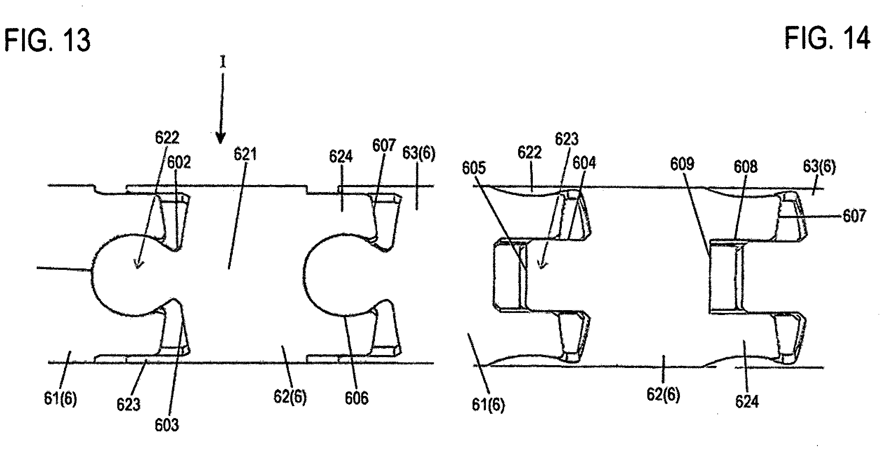

Der genauere Aufbau des Abwinkelungsabschnittes A ist in den Figuren 13 bis 18 gezeigt.

Der Abwinkelungsabschnitt A hat einzelne Gelenkglieder 6, die in Längsrichtung des Abwinkelungsabschnittes A angeordnet sind. Die einzelnen Gelenkglieder 6 sind relativ zueinander schwenkbar. In den Figuren 13 und 14 sind drei hintereinander angeordnete Gelenkglieder 6 gezeigt: ein Gelenk 61, proximal vom Gelenk 61 ein Gelenk 62 und proximal vom Gelenk 62 ein Gelenk 63.

Die Gelenkglieder 6 sind zueinander gleich gestaltet mit Ausnahme des am weitesten distal befindlichen Gelenkglieds 6 und des am weitesten proximal befindlichen Gelenkglieds 6.

Der Aufbau des jeweiligen Gelenkglieds 6 ist nachstehend anhand Gelenkglied 62 erörtert.

Das Gelenkglied 62 ist als ein Rohrabschnitt des genannten Rohrelements durch Laserschneiden ausgebildet. Das Gelenkglied 62 besitzt am Umfang des Rohrelements distale Begrenzungslinien 601, 602, 603, 604 und 605 und proximale Begrenzungslinien 606, 607, 608 und 609.

Die einzelnen distalen Begrenzungslinien setzen sich zusammen aus einer kreisartig geformten Kopflinie 601, zwei Halslinien 602, zwei Schulterlinien 603, zwei Armlinien 604 und einer Armendlinie 605. Genauer gesagt ist die distale Seite des Gelenkglieds 62 folgendermaßen gebildet. Die kreisartig geformte Kopflinie 601 bildet einen unvollständigen Kreis, der an der proximalen Seite an jeder Seite in eine Halslinie 602 übergeht. An jeder der beiden Halslinien 602 schließt sich eine Schulterlinie 603 an, die annähernd senkrecht zur Achse des Rohrelements verläuft. An jeder der beiden Schulterlinien 603 schließt sich eine Armlinie 604 an, die annähernd parallel zur Achse des Rohrelements in die distale Richtung verläuft. Die beiden distalen Enden der Armlinien 604 sind durch eine Armendlinie 605 verbunden, die wieder senkrecht zur Achse des Rohrelements verläuft.

Dadurch hat das Gelenkglied 62 einen Hauptkörper 621, von dem zur distalen Seite hin ein erster Kopf 622, ein erster Arm 623, ein zweiter Kopf 622 und ein zweiter Arm 623 jeweils um 90 Grad entlang einer gedachten Umfangslinie, die senkrecht zur Achse des Gelenkglieds 62 verläuft, vorragen. Somit erstrecken sich die Köpfe 622, 622 in einer ersten gedachten Ebene. Die Arme 623, 623 erstrecken sich in einer zweiten gedachten Ebene, die um 90 Grad versetzt zur ersten gedachten Ebene ist. Die beiden Köpfe 622, 622 des Gelenkgliedes 62 bilden eine Schwenkachse für das distal von ihnen befindliche Gelenkglied 61.

Jeder Kopf 622 ist an der distalen Seite durch eine Kopflinie 601 gebildet. Zwischen dem Kopf 622 und dem Hauptkörper 621 ist eine Verengung durch die Halslinien 602 gebildet. Der jeweilige Kopf 622 ragt weiter in der distalen Richtung vor als der jeweilige Arm 623.

Die einzelnen proximalen Begrenzungslinien setzen sich zusammen aus einer gebogenen Fußlinie 606, zwei Bodenlinien 607, zwei geraden Fußlinien 608 und einer Bauchlinie 609. Genauer gesagt ist die proximale Seite des Gelenkglieds 62 folgendermaßen gebildet. Die gebogene Fußlinie 606 bildet einen unvollständigen Kreis, der an der proximalen Seite offen ist. An den offenen Enden des unvollständigen Kreises geht die gebogene Fußlinie 606 jeweils in die Bodenlinie 607 über, die jeweils annähernd senkrecht zur Achse des Rohrelements verläuft.

An jeder der beiden Bodenlinien 607 schließt sich eine gerade Fußlinie 608 an, die annähernd parallel zur Achse des Rohrelements in die distale Richtung verläuft. Die beiden distalen Enden der geraden Fußlinien 608 sind durch eine Bauchlinie 609 verbunden, die wieder senkrecht zur Achse des Rohrelements verläuft.

Dadurch hat das Gelenkglied 62 an der proximalen Seite des Hauptkörpers 621 zwei Füsse 624, die sich in proximaler Richtung erstrecken. Jeder Fuß 624 hat in Erstreckungsrichtung eine gerade Seite an der geraden Fußlinie 608 und eine gekrümmte Seite an der gebogenen Fußlinie 606.

In dem Bereich zwischen den beiden geraden Fußlinien 608 ist ein Arm des proximal befindlichen Gelenkgliedes 63 in Längsrichtung verschiebbar angeordnet. In dem Bereich zwischen den beiden gebogenen Fußlinien 606 wird ein Kopf des proximal befindlichen Gelenkgliedes 63 in Längsrichtung unbeweglich gehalten. Allelfalls eine geringfügige Bewegung aufgrund eines Spieles zwischen dem Innenumfang der gebogenen Fußlinie und dem Außenumfang der kreisartig geformten Kopflinie ist möglich.

Im nicht gebogenen Zustand des Abwinkelungsabschnittes A ist die Bauchlinie 609 von der Armendlinie 605 des proximal befindlichen Gelenkgliedes 63 beabstandet, wie dies in Fig. 14 gezeigt ist. Die Armendlinie 605 und die Bauchlinie 609 des proximal befindlichen Gelenkgliedes 63 sind parallel zueinander.

Im nicht gebogenen Zustand des Abwinkelungsabschnittes A ist die Bodenlinie 607 von der Schulterlinie 603 des proximal befindlichen Gelenkgliedes 63 beabstandet, wie dies in Fig. 14 gezeigt ist. Die Bodenlinie 607 und die Schulterlinie 603 des proximal befindlichen Gelenkgliedes 63 können parallel zueinander oder annähernd parallel zueinander sein oder leicht winklig zueinander sein, wie dies in Fig. 14 gezeigt ist. Zwischen der Bodenlinie 607 und der Schulterlinie 603 des proximal befindlichen Gelenkgliedes 63 ist nicht nur eine einfache Schnittlinie erzeugt worden, sondern das Material des Rohrelementes ist als ein viereckiges Stück herausgeschnitten worden.

Ein jeweiliger Kopf 622 bildet einen Kupplungsabschnitt, der mit einem benachbarten Gelenkglied 6 gekuppelt ist. Die Füße 624 bilden einen Führungsabschnitt, der mit einem benachbarten Gelenkglied 6 so in Eingriff steht, dass eine axiale Bewegung der Gelenkglieder 6 zueinander ermöglicht ist.

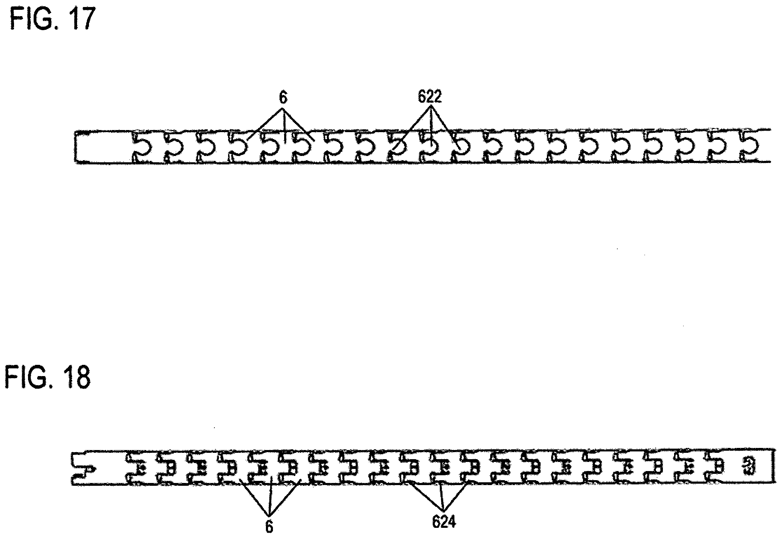

Fig. 17 zeigt eine Draufsicht auf den Abwinkelungsabschnitt A mit den jeweiligen Gelenkgliedern 6. In der Draufsicht sind die Köpfe 622 der Gelenkglieder 6 zu sehen.

Fig. 18 zeigt eine Seitenansicht des Abwinkelungsabschnittes A mit den jeweiligen Gelenkgliedern 6. In der Seitenansicht sind die Füße 624 der Gelenkglieder 6 zu sehen.

Das am weitesten distal befindliche Gelenkglied 6 hat keinen Kopf und ist in den Figuren 2 und 17 bis 21 gezeigt.

Das am weitesten proximal befindliche Gelenkglied 6 hat keinen Fuß und ist in den Figuren 2, 11 und 18 gezeigt.

Im Ausführungsbeispiel kann der Abwinkelungsabschnitt A in zwei Abwinkelungsrichtungen abgewinkelt werden, nämlich in den Figuren 13 und 14 (und Fig. 17) nach oben und unten, wobei die jeweiligen Köpfe 622 der Gelenkglieder 6 Biegeachsen der Gelenkglieder 6 bilden. Anders ausgedrückt, ist der Abwinkelungsabschnitt A in Fig. 17 nach oben und unten schwenkbar. In der Darstellung von Fig. 18 ist der Abwinkelungsabschnitt A zum Betrachter hin und vom Betrachter schwenkbar.

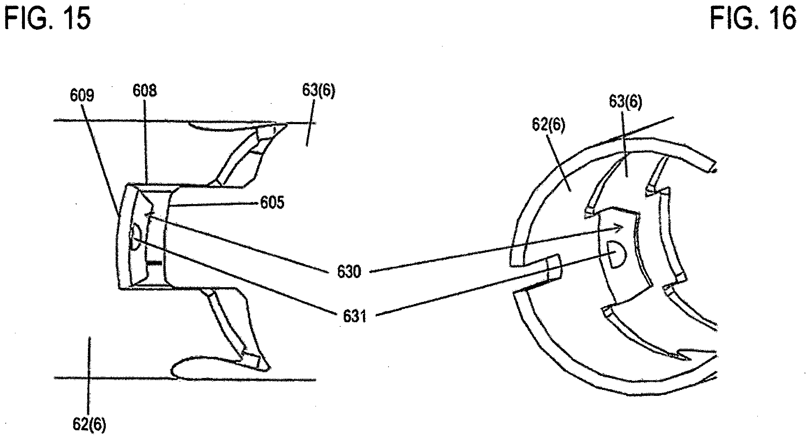

Wie dies in den Figuren 15 und 16 gezeigt ist, bildet die Bauchlinie 609 einen Scharnierabschnitt für eine Seilführungslasche 630. Die Seilführungslasche 630 erstreckt sich von der Bauchlinie 609. Für die Seilführungslasche 630 wird ein Materialabschnitt genommen, der sich entlang der geraden Fußlinien 608 bis zur Armendlinie 605 des proximal befindlichen Gelenkgliedes 63 erstreckt. Die Seilführungslasche 630 ist an der Bauchlinie 609 angelenkt und um 90 Grad nach innen gebogen. Die Seilführungslasche 630 besitzt ein zentrisches Loch 631. Das Loch 631 hat einen größeren Durchmesser als der Steuerdraht.

Jedes der Gelenkglieder 6 besitzt die Seilführungslaschen 630 mit dem Loch 631 so, dass die Seilführungslaschen 630 für einen spezifischen Steuerdraht in Längsrichtung des Abwinkelungsabschnittes A hintereinander angeordnet sind. Die Seilführungslaschen 630 dienen als Führungsvorsprünge, an denen ein Steuerdraht abgestützt ist Somit führen die Seilführungslaschen 630 den ihnen zugewiesenen Steuerdraht durch den Abwinkelungsabschnitt A.

Die Gelenkglieder 6 können am Abwinkelungsabschnitt A so angeordnet sein, dass ihre Köpfe in die proximale Richtung weisen, wie dies in Fig. 17 gezeigt ist. Alternativ können die Gelenkglieder 6 am Abwinkelungsabschnitt A so angeordnet sein, dass ihre Köpfe in die distale Richtung weisen, wie dies in Fig. 13 angedeutet ist.

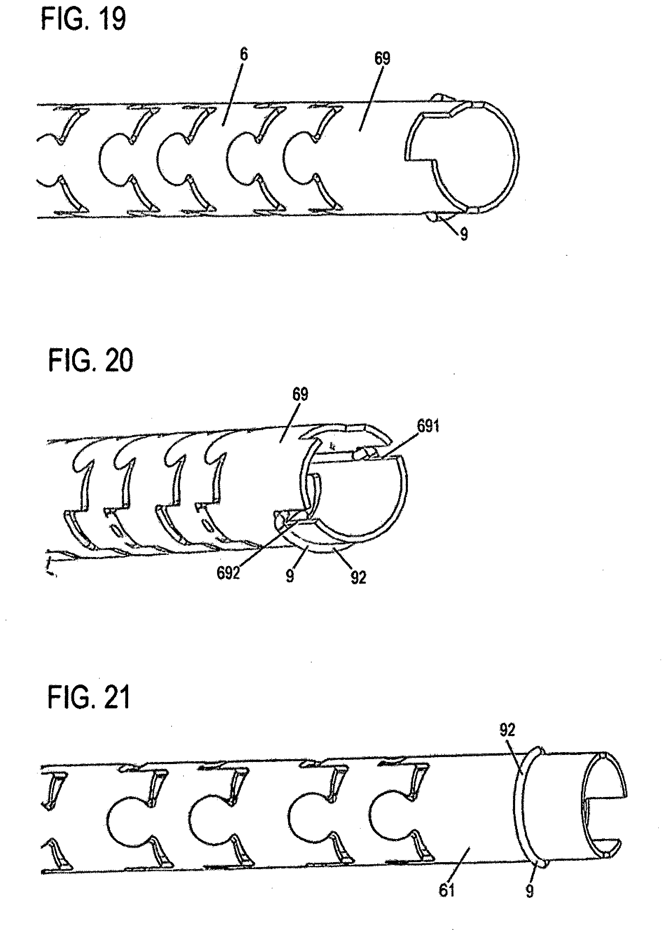

Das distale Ende des Abwinkelungsabschnittes A ist in den Fig. 19 bis 21 gezeigt. In den in den Fig. 19 bis 21 ist das am weitesten an der distalen Seite befindliche Gelenkglied 69 des Abwinkelungsabschnittes A erkennbar. In diesem am weitesten an der distalen Seite befindlichen Gelenkglied 69 ist die distale Seite des Steuerdrahtes 9 verankert. Der Steuerdraht 9 erstreckt sich vom Kontrollkörper 3 bis zum am weitesten an der distalen Seite befindlichen Gelenkglied 69 des Abwinkelungsabschnittes A.

### Befestigung des Steuerdrahtes

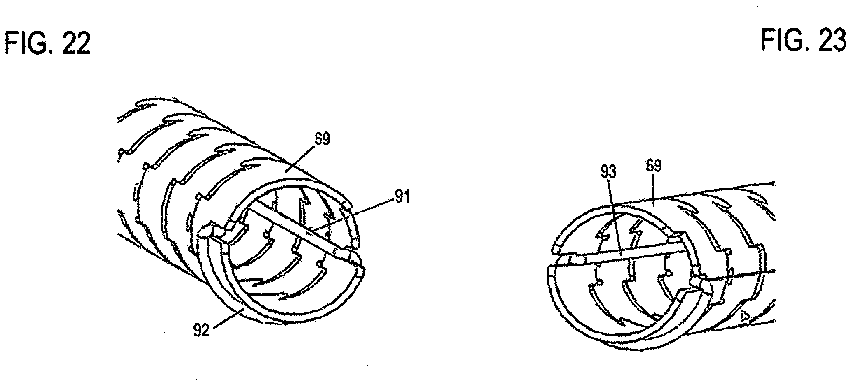

Die genaue Befestigung des Steuerdrahtes 9 ist in den Figuren 22 und 23 gezeigt.

Der Steuerdraht 9 ist im Kontrollkörper 3 am Steuerrad G befestigt. Wenn das Steuerrad G in eine Spannrichtung gedreht wird, wird der Steuerdraht 9 gespannt. Wenn das Steuerrad G in die zur Spannrichtung entgegengesetzte Entlasungsrichtung gedreht wird, wird der Steuerdraht 9 entlastet.

Der Steuerdraht 9 erstreckt sich vom Kontrollkörper 3 kommend im Einführschlauch 2 verlaufend bis zum Gelenkglied 69 und bildet einen ersten Abschnitt 91. Dieser erste Abschnitt 91 des Steuerdrahtes 9 läuft am Innenumfang des Einführschlauches 2 entlang. Dieser erste Abschnitt 91 des Steuerdrahtes 9 ist anhand Bezugszeichen 91 in Fig. 22 gezeigt. An der distalen Seite des Gelenkgliedes 69 ist ein die Umfangswand des Gelenkgliedes 69 durchdringender Schlitz 691 ausgebildet (siehe Fig. 20), der sich in der Längsrichtung des Gelenkgliedes 69 erstreckt. Ein weiterer ähnlicher Schlitz 692 ist an der distalen Seite des Gelenkgliedes 69 dem Schlitz 691 diametral gegenüberliegend vorgesehen.

Der Steuerdraht 9 erstreckt sich am Innenumfang des Gelenkgliedes 69 in die distale Richtung und durchdringt den Schlitz 691 nach außen, ist am Außenumfang des Gelenkgliedes 69 in Umfangsrichtung des Gelenkgliedes 69 bis zum Schlitz 692 gewunden, durchdringt den Schlitz 692 nach innen, und erstreckt sich am Innenumfang des Gelenkgliedes 69 in die proximale Richtung bis hin zum Steuerrad G im Kontrollkörper 3.

Der Steuerdraht 9 ist somit in einen ersten Abschnitt 91, der sich vom Steuerrad G im Kontrollkörper 3 bis zum Schlitz 691 erstreckt, einen zweiten Abschnitt 92, der sich vom Schlitz 691 am Außenumfang des Gelenkgliedes 69 in Umfangsrichtung des Gelenkgliedes 69 bis zum Schlitz 692 erstreckt, und einen dritten Abschnitt 93 geteilt, der sich vom Schlitz 692 bis zum Steuerrad G im Kontrollkörper 3 erstreckt.

Durch Drehen des Steuerrades G in die Spannrichtung wird der Steuerdraht 9 gespannt und somit der Abwinkelungsabschnitt A abgewinkelt, da der am Gelenkglied 69 verankerte dritte Abschnitt 93 in die proximale Richtung gedrängt wird. Der dritte Abschnitt 93 des Steuerdrahtes 9 bildet somit einen distalen Verankerungsabschnitt des Steuerdrahtes 9.

### Herstellverfahren

Der Einführschlauch 2 kann durch ein einziges Rohrelement hergestellt werden, das per Laser geschnitten wird. Das Rohrelement ist aus einem relativ harten Material, wie z.B. Edelstahl oder auch geeigneter harter Kunststoff hergestellt. Durch die Schnitte wird das zunächst harte Rohrelement flexibel, behält aber seine Steifigkeit.

Die Schnitte erzeugen die jeweiligen seitlichen Einschnitte (senkrecht zur Achse laufende Schnitte) 98, 99 im proximalen passiven flexiblen Abschnitt 20, das Loch 77, den Schnitt 70 im Übergangsbereich K, das Loch 631, die jeweiligen Gelenkglieder 6 im distalen Abwinkelungsabschnitt A und die Schlitze 691, 692. Diese Reihenfolge ist nicht als Einschränkung aufzufassen. Z.B. können die Schlitze 691, 692 vor den Gelenkgliedern 6 geschnitten werden. Außerdem kann die Reihenfolge der Schnitte auch umgekehrt werden.

Die Flexibilität und auch die Steifigkeit des Rohrelementes können anhand der Form, der Anordnung und der Größe der Schnitte gesteuert werden.

Der Ort der jeweiligen Schnitte kann zuvor berechnet und vorbestimmt werden. In einer programmierbaren Laserschneidmaschine können die vorgegebenen Daten für die jeweiligen Schnitte eingegeben werden, um den Einführschlauch 2 automatisch zu erzeugen.

Die einzelnen Gelenkglieder 6 werden vollständig ausgeschnitten und bilden voneinander körperlich getrennte Körper, die lediglich formschlüssig verbunden sind.

Nach dem Laserschneiden des Rohrelementes werden die Laschen 72 und die Seilführungslaschen 630 nach innen gebogen. Somit ist der Rohkörper für den Einführschlauch 2 fertiggestellt.

In diesem Rohkörper für den Einführschlauch 2 kann nun der Steuerdraht 9 eingeführt und befestigt werden. Der Rohkörper für den Einführschlauch 2 kann am Kontrollkörper 3 befestigt werden. Ferner kann auf den Rohkörper für den Einführschlauch 2 ein den Rohkörper für den Einführschlauch 2 umgebender Überzug aus vorzugsweise Metall zur Abschirmung der elektrischen Steuerung und auf diesen ein elastischer Mantel aus Kunststoff oder Gummi aufgezogen werden. Der elastische Mantel aus Kunststoff oder Gummi kann einem thermischen Schrumpfen ausgesetzt werden.

### Zweites Ausführungsbeispiel

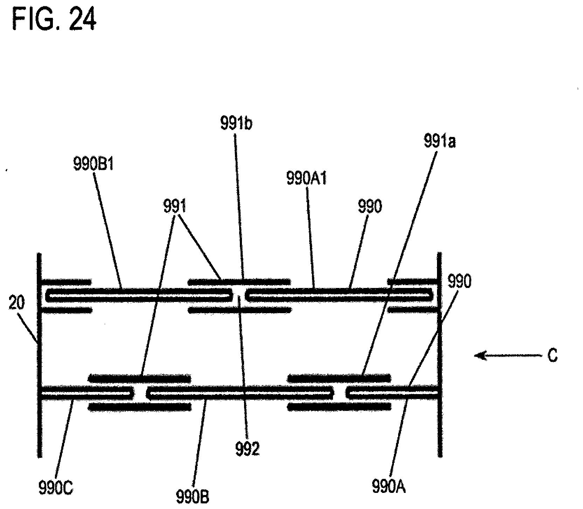

Nachstehend ist unter Bezugnahme auf Fig. 24 ein zweites Ausführungsbeispiel beschrieben.

Fig. 24 zeigt eine ausschnittartige schematische Darstellung des proximalen passiven flexiblen Abschnittes, das im zweiten Ausführungsbeispiel angewendet ist.

Der gemäß dem in Fig. 24 dargestellten Prinzip aufgebaute proximale passive flexible Abschnitt 20 kann den proximalen passiven flexiblen Abschnitt 20 aus dem ersten Ausführungsbeispiel ersetzen. Anders ausgedrückt sind der Kontrollkörper 3 und der Abwinkelungsabschnitt A mit dem proximalen passiven flexiblen Abschnitt 20 des vorliegenden zweiten Ausführungsbeispiels kombinierbar.

Wie vorstehend beschrieben, sind der distale Abwinkelungsabschnitt A und der proximale passive flexiblen Abschnitt 20 mit den drei Zonen B, C und D aus einem einzigen Rohr oder Schlauch gebildet, siehe auch Fig. 1.

Die Zone B bildet einen Übergangsbereich B zwischen dem mittleren Bereich C und dem Abwinkelungsabschnitt A. Die Zone C bildet den mittleren Bereich C. Die Zone D bildet einen Anschlussbereich D des proximalen passiven flexiblen Abschnittes 20 am Kontrollkörper 3. Anders ausgedrückt ist der gesamte Einführschlauch inklusive dem Anschlussbereich D am Kontrollkörper 3, dem mittleren Bereich C, dem Übergangsbereich B zwischen dem mittleren Bereich C und dem Abwinkelungsabschnitt A, und dem Abwinkelungsabschnitt A aus einem einzigen Rohrelement hergestellt.

Fig. 1 zeigt den proximalen passiven flexiblen Abschnitt 20 der besseren Übersichtlichkeit so, als wenn die drei Zonen B, C und D entlang der Längsrichtung des Einführschlauches 2 zueinander gleich lang wären. Dies ist natürlich nicht der Fall. Der mittlere Bereich C ist länger als der Übergangsbereich B und der Anschlussbereich D. Der mittlere Bereich C ist im proximalen passiven flexiblen Abschnitt 20 am längsten. Anders ausgedrückt wird der eigentliche proximale passive flexible Abschnitt 20 durch den Aufbau des mittleren Bereiches C gebildet. Die Biegeeigenschaften, die Elastizität und der Torsionswiderstand des proximalen passiven flexiblen Abschnittes 20 werden durch den Aufbau des mittleren Bereiches C verwirklicht.

Nachstehend ist der Aufbau des mittleren Bereiches C des proximalen passiven flexiblen Abschnittes 20 anhand Fig. 24 genauer beschrieben.

Der proximale passive flexible Abschnitt 20 ist aus dem vorstehend bereits beschriebenen Rohrelement hergestellt. Im mittleren Bereich C sind durch Laserschneiden entlang der Längsrichtung des Rohrelements eine Vielzahl an Hauptschnitten 990 geschnitten. Diese Hauptschnitte 990 verlaufen zueinander parallel. Die Hauptschnitte 990 verlaufen senkrecht zur Achse des Rohrelements.

Genauer gesagt verlaufen die Hauptschnitte 990 entlang des Umfangs des mittleren Bereiches C in unterbrochener Weise so, dass nicht geschnittene Stege 992 zwischen auf einer Umfangslinie liegenden Hauptschnitt-abschnitten belassen sind. Im vorliegenden Ausführungsbeispiel sind in Umfangsrichtung gesehen vier Hauptschnitt-abschnitte ausgebildet.

Fig. 24 zeigt diese Hauptschnitt-abschnitte genauer. Fig. 24 zeigt eine erste Abfolge von in Umfangsrichtung gebildeten Hauptschnitt-abschnitten mit den Bezugszeichen 990A, 990B und 990C. Fig. 24 zeigt ausserdem eine zweite Abfolge von in Umfangsrichtung gebildeten Hauptschnitt-abschnitten mit den Bezugszeichen 990A1 und 990B1. Die erste Abfolge von Hauptschnitt-abschnitten mit den Bezugszeichen 990A, 990B und 990C ist in Längsrichtung benachbart zur zweiten Abfolge von in Umfangsrichtung gebildeten Hauptschnitt-abschnitten mit den Bezugszeichen 990A1 und 990B1. Die Länge der Hauptschnitt-abschnitte in Umfangsrichtung ist stets gleich. Das heißt, nicht nur die Länge der Hauptschnitt-abschnitte in Umfangsrichtung einer bestimmten Abfolge von Hauptschnitt-abschnitten ist zueinander gleich, sondern die die Länge der Hauptschnitt-abschnitte in Umfangsrichtung aller Abfolgen von Hauptschnitt-abschnitten im gesamten mittleren Bereich C ist zueinander gleich.

In der in Fig. 24 gezeigten ersten Abfolge von Hauptschnitt-abschnitten sind ein erster Hauptschnitt-abschnitt 990A, ein zweiter Hauptschnitt-abschnitt 990B und ein dritter Hauptschnitt-abschnitt 990C gezeigt. Ein nicht sichtbarer vierter Hauptschnitt-abschnitt befindet sich an der dem Betrachter abgewandten Seite des Rohrelementes hinter der Zeichnungsebene. Der erste Hauptschnitt-abschnitt 990A, der zweite Hauptschnitt-abschnitt 990B, der dritte Hauptschnitt-abschnitt 990C und der nicht gezeigte vierte Hauptschnitt-abschnitt sind in Umfangsrichtung des Rohrelementes aufeinanderfolgend ausgebildet. Somit ist das Rohrelement an dieser Umfangslinie vier mal in gleicher Länge abschnittsweise geschnitten. Ein jeweiliger Steg 992 ist zwischen einem Ende des ersten Hauptschnitt-abschnittes 990A und einem Anfang des zweiten Hauptschnitt-abschnittes 990B, einem Ende des zweiten Hauptschnitt-abschnittes 990B und einem Anfang des dritten Hauptschnitt-abschnittes 990C, einem Ende des dritten Hauptschnitt-abschnittes 990C und einem Anfang des nicht gezeigten vierten Hauptschnitt-abschnittes, und einem Ende des nicht gezeigten vierten Hauptschnitt-abschnittes und einem Anfang des ersten Hauptschnitt-abschnittes 990A belassen. An diesem Bereich des Steges 992 ist das Rohrelement nicht geschnitten.

In der in Fig. 24 gezeigten zweiten Abfolge von Hauptschnitt-abschnitten sind ein erster Hauptschnitt-abschnitt 990A1 und, ein zweiter Hauptschnitt-abschnitt 990B1 gezeigt. Ein nicht sichtbarer dritter Hauptschnitt-abschnitt und ein nicht sichtbarer vierter Hauptschnitt-abschnitt befinden sich an der dem Betrachter abgewandten Seite des Rohrelementes hinter der Zeichnungsebene.

Die Hauptschnitt-abschnitte der zweiten Abfolge sind relativ zu den Hauptschnitt-abschnitten der ersten Abfolge versetzt angeordnet. Der Bereich der ersten Abfolge, an dem die Hauptschnitt-abschnitte 990A, 990B und 990C den jeweiligen Steg 992 belassen, entspricht in der benachbarten zweiten Abfolge einem Bereich, der die in Umfangsrichtung des Rohrelements gesehen die Mitte des Hauptschnitt-abschnittes 990A1 und 990B1 bildet. Somit sind die Stege von Abfolge zu Abfolge der Hauptschnitte 990 in Längsrichtung des Rohrelements um 45 Grad versetzt positioniert.

Die Schneidbreite sämtlicher Hauptschnitte 990 im Rohrelement ist gleich. Der Abstand sämtlicher Abfolgen der Hauptschnitte 990 im Rohrelement ist zueinander gleich.

In Längsrichtung des Rohrelements ist benachbart zu einem jeden Steg 992 jeweils ein Nebenschnitt 991 vorgesehen, wie dies Fig. 24 zeigt.

An beiden Seiten in Längsrichtung des Rohrelements ist benachbart zum Steg 992 ein Nebenschnitt 991 ausgebildet. Der Nebenschnitt 991 ist kürzer als der Hauptschnitt 990. Der Nebenschnitt 991 überlappt mit den Enden der benachbarten Hauptschnitte 990.

Sämtliche Nebenschnitte 991 haben zueinander die gleiche Länge in Umfangsrichtung des Rohrelements. Sämtliche Nebenschnitte 991 sind parallel zueinander und auch parallel zu den Hauptschnitten 990.

Einer Abfolge an Hauptschnitten 990 sind benachbart an beiden Seite in Längsrichtung des Rohrelements je eine Abfolge an Nebenschnitten 991 zugeordnet. Anders ausgedrückt hat jede Abfolge an Hauptschnitten 990 eine proximale Abfolge an Nebenschnitten 991 und eine distale Abfolge an Nebenschnitten 991.

Somit folgt, entlang der Längsrichtung des Rohrelements gesehen, einer Abfolge an Hauptschnitten 990 eine distale Abfolge an Nebenschnitten 991, der wieder eine proximale Abfolge an Nebenschnitten 991 der nächsten Abfolge an Hauptschnitten 990 folgt. Entlang der Längsrichtung des Rohrelements gesehen hat eine Abfolge an Nebenschnitten 991 an einer Seite eine weitere Abfolge an Nebenschnitten 991 als Nachbar und an der anderen Seite eine Abfolge an Hauptschnitten 990 als Nachbar.

Die Nebenschnitte 991 sind in Längsrichtung des Rohrelements näher zu den nächsten Hauptschnitten 990 als zu den nächsten Nebenschnitten 991 ausgebildet.

Anders ausgedrückt sind benachbart zu den Hauptschnitten 990 Nebenschnitte 991 so vorgesehen, dass sie zu den benachbarten Hauptschnitten 990 näher angeordnet sind als zu den benachbarten Nebenschnitten 991.

Um dies zu verdeutlichen, zeigt Fig. 24 die Nebenschnitte 991 für die erste Abfolge von Hauptschnitt-abschnitten als Nebenschnitte 991a und die Nebenschnitte 991 für die zweite Abfolge von Hauptschnitt-abschnitten als Nebenschnitte 991b. Die Nebenschnitte 991a für die erste Abfolge von Hauptschnitt-abschnitten sind zu den benachbarten Hauptschnitt-abschnitte 990A, 990B und 990C näher angeordnet als zu den benachbarten Nebenschnitten 991b. Somit sind benachbarte Schnitte im Rohrelement ungleich beabstandet.

Die Schneidbreite sämtlicher Nebenschnitte 991 im Rohrelement ist gleich. Die Schneidbreite der Nebenschnitte 991 ist schmaler als die Schneidbreite der Hauptschnitte 990.

### Effekt des zweiten Ausführungsbeispiels

Wie im ersten Ausführungsbeispiel stellt der Aufbau des zweiten Ausführungsbeispiels einen Einführschlauch 2 mit einer sehr hohen Flexibilität und gleichzeitig einem hohen Torsionswiderstand sicher.

### Weitere Alternativen

Im ersten und zweiten Ausführungsbeispiel hat der flexible Abschnitt 20 unter Betrachtung in proximaler Richtung eine erste Zone B, eine zweite Zone C und eine dritte Zone D mit unterschiedlicher Flexibilität. Die Anzahl an Zonen oder Bereichen mit unterschiedlicher Flexibilität ist nicht beschränkt. Der flexible Abschnitt 20 kann auch mehr oder weniger Zonen mit unterschiedlicher Flexibilität haben. Das Prinzip ist auch auf einen Einführschlauch anwendbar, bei dem der flexible Abschnitt 20 eine durchgehend gleichbleibende Flexibilität besitzt.

Im ersten und zweiten Ausführungsbeispiel ist das Rohrelement des Einführschlauches 2 aus Edelstahl gebildet.

Das Material des Einführschlauches 2 kann ein beliebiges ausreichend steifes Material sein, wie z.B. ein steifer Kunststoff. In einer weiteren Alternative kann Nitinol (eine Nickel-Titan-Legierung) als Rohrmaterial angewendet werden. Dieses Material hat u.a. die Eigenschaft einer sogenannten Superelastizität, d.h. es kann in weiten Bereichen elastisch verformt werden, ohne sich bleibend zu verbiegen.

Im ersten und zweiten Ausführungsbeispiel werden durch eine Laserschneidmaschine im Rohrelement Schnitte vorgesehen. Diese Schnitte können sehr präzise vorgesehen werden. Daher wird eine Herstellung per Laser bevorzugt. Im Prinzip ist es jedoch denkbar, dass diese Schnitte auch durch andere Herstellverfahren wie z.B. Sägen, Drahtsägen etc. gefertigt werden.

Im ersten und zweiten Ausführungsbeispiel kann der Abwinkelungsabschnitt A in zwei Abwinkelungsrichtungen abgewinkelt werden, nämlich in den Figuren 6 und 7 nach oben und unten. In einer Alternative können die einzelnen Gelenkglieder 6 so ausgebildet sein, dass ihre Köpfe 622 von Gelenkglied 6 zu Gelenkglied 6 um 90 Grad gedreht um die Achse des Abwinkelungsabschnittes A (Achse der Gelenkglieder 6) versetzt sind. In dieser Alternative kann der Abwinkelungsabschnitt A in vier Abwinkelungsrichtungen abgewinkelt werden, nämlich in den Figuren 6 und 7 nach oben und unten und zum Betrachter hin und vom Betrachter weg.

In der Alternative, in der der Abwinkelungsabschnitt A in vier Abwinkelungsrichtungen abgewinkelt werden kann, können zwei Steuerdrähte 9 verwendet werden, die um 90 Grad zueinander versetzt im Einführschlauch 2 verlaufen. Das Gelenkglied 92 ist dann mit vier distalen Schlitzen versehen, die ebenfalls um 90 Grad zueinander versetzt sind.

Im Ausführungsbeispiel ist ein jeweiliges Gelenkglied 6 in der beschriebenen Form ausgebildet. Die Erfindung ist nicht auf die Form des Gelenkgliedes 6 beschränkt. Es ist ausreichend, wenn im Abwinkelungsabschnitt A Gelenkglieder geschnitten werden, die miteinander gekuppelt sind und eine Auslenkbewegung des Abwinkelungsabschnittes A ermöglichen.

Der gemäß dem in Fig. 24 dargestellten Prinzip aufgebaute proximale passive flexible Abschnitt C kann im ersten oder zweiten Ausführungsbeispiel angewendet werden. Dies bedeutet, dass der in Fig. 24 dargestellte proximale passive flexible Abschnitt C einen Teil des einstückigen Rohrelementes für den gesamten Einführschlauch 20 bildet. Das Rohrelements für den gesamten Einführschlauch 20 wird somit inklusive dem proximalen passiven flexiblen Abschnitt C aus einem Rohrelement durch Laserschneiden hergestellt.

Alternativ kann der proximale passive flexible Abschnitt C im ersten oder zweiten Ausführungsbeispiel separat vom restlichen Einführschlauch 20 hergestellt werden.

Im Ausführungsbeispiel von Fig. 24 sind in Längsrichtung des Rohrelements jeweils zwei Nebenschnitte benachbart zu dem Steg an beiden Seiten des Steges angeordnet. In einer Alternative kann in Längsrichtung des Rohrelements ein Nebenschnitt benachbart zu dem Steg an einer Seite des Steges jeweils angeordnet sein.

Im ersten Ausführungsbeispiel sind die Hauptschnitte so vorgesehen, dass entlang des Umfangs des Rohrelements zwischen den Hauptschnitt-Abschnitten zwei Stege verbleiben.

Im zweiten Ausführungsbeispiel sind die Hauptschnitte so vorgesehen, dass entlang des Umfangs des Rohrelements zwischen den Hauptschnitt-Abschnitten vier Stege verbleiben.

Die Erfindung ist nicht darauf beschränkt. Vorzugsweise beträgt die Anzahl an Stegen entlang des Umfangs des Rohrelements zwischen den Hauptschnitt-Abschnitten mindestens zwei oder mehr und kann beliebig hoch sein.

Im ersten Ausführungsbeispiel ist die Schnittbreite der Hauptschnitte 98 größer als die Schnittbreite der Nebenschnitte 99. Auch im zweiten Ausführungsbeispiel kann die Schnittbreite der Hauptschnitte größer als die Schnittbreite der Nebenschnitte sein. Das Prinzip der Erfindung ist aber auch bei dem Fall anwandbar, bei dem die Schnittbreite der Hauptschnitte gleich der Schnittbreite der Nebenschnitte ist.

Die Erfindung ist bei einem Duodenoskop, einem Gastroskop, einem Colonoskop oder einem ähnlichen Endoskop vorteilhaft anwendbar. Das Prinzip der Erfindung kann auch bei einer beliebigen anderen Art an Endoskop angewendet werden.

### Bezugszeichenliste

- 1: Endoskop
- 2: Einführschlauch, Rohr
- 3: Kontrollkörper
- 6: Gelenkglied
- 8: Führungsfeder
- 9: Steuerdraht
- 20: flexibler Abschnitt
- 61: Gelenkglied
- 62: Gelenkglied
- 63: Gelenkglied
- 69: am weitesten an der distalen Seite befindliches Gelenkglied
- 70: Schnitt
- 71: Scharnier
- 72: Lasche
- 73: unteres Ohr
- 74: oberes Ohr 74
- 75: Laschenmittelstück
- 77: Loch
- 91: erster Abschnitt des Steuerdrahtes
- 92: zweiter Abschnitt des Steuerdrahtes
- 93: dritter Abschnitt des Steuerdrahtes
- 97: Steg
- 98: Hauptschnitt
- 99: Nebenschnitt
- 201: Schnitt von oben
- 202: Schnitt von unten
- 203: nicht geschnittener Zwischenraum
- 204: Schnitt von der Seite
- 601: Kopflinie
- 602: Halslinie
- 603: Schulterlinie
- 604: Armlinie
- 605: Armendlinie
- 606: gebogene Fußlinie
- 607: Bodenlinie
- 608: gerade Fußlinie
- 609: Bauchlinie
- 621: Hauptkörper
- 622: Kopf
- 623: Arm
- 624: Fuß
- 630: Seilführungslasche
- 631: zentrisches Loch
- 691: Schlitz
- 692: Schlitz
- 801: Schnitt von oben
- 802: Schnitt von unten
- 803: nicht geschnittener Zwischenraum
- 805: Ringabschnitt mit kurzen Schnitten
- 811: kurzer Schnitt von oben
- 812: kurzer Schnitt von unten
- 880: Seilführungslasche
- 990: Hauptschnitt
- 991: Nebenschnitt
- 992: Steg
- 1000: Einführschlauch
- 1001: Metallblattspirale
- 1002: Metallblattspirale
- 1003: Metallgeflecht
- 1004: Kunststoffüberzug
- A: Abwinkelungsabschnitt
- A': Abwinkelungsabschnitt
- B: erste Zone (distaler Bereich)
- C: zweite Zone (mittlerer Bereich)
- D: dritte Zone (proximaler Bereich)
- F: erstes Steuerrad (erstes Steuerelement)
- G: zweites Steuerrad (zweites Steuerelement)
- H: Abstand
- J: Kontrollkörpergehäuse
- K: Übergangsbereich
- L: Längslinie des Rohres 2
- M: Abstand
- N: Abstand
- X: Bereich, der für die Entstehung einer hohen Flexibilität des Rohres 2 zuständig ist
- Y: Bereich, der für die Entstehung eines hohen Torsionswiderstandes des Rohres 2 zuständig ist

## Patentansprüche

1. Verfahren zur Herstellung eines Einführschlauches (2) eines Endoskops aus einem Rohrelement,
wobei der Einführschlauch (2) einen proximalen passiven flexiblen Abschnitt (C) und einen distalen Abwinkelungsabschnitt (A) aufweist,
wobei im proximalen passiven flexiblen Abschnitt (C) Schnitte (98, 99; 990, 991) vorgesehen werden, um ein Biegen des proximalen passiven flexiblen Abschnittes (C) zu ermöglichen,
wobei die Schnitte (98, 99; 990, 991) im proximalen passiven flexiblen Abschnitt (C) so ausgebildet werden, dass benachbarte Schnitte (98, 99; 990, 991) ungleich beabstandet sind;
**dadurch gekennzeichnet, dass**
im proximalen passiven flexiblen Abschnitt (C) Hauptschnitte (98; 990) vorgesehen werden, die in Längsrichtung des proximalen passiven flexiblen Abschnittes (C) zueinander gleich beabstandet (H) sind, und
benachbart zu den Hauptschnitten (98; 990) Nebenschnitte (99; 991) im proximalen passiven flexiblen Abschnitt (C) vorgesehen werden, die in Längsrichtung des proximalen passiven flexiblen Abschnittes (C) zu den benachbarten Hauptschnitten (98; 990) an einer Seite der Nebenschnitte (99; 991) näher angeordnet (N) sind als zu den benachbarten Hauptschnitten (98; 990) an der anderen Seite der Nebenschnitte (99; 991).

2. Verfahren gemäß Anspruch 1, wobei
die Hauptschnitte (98; 990) parallel zueinander geschnitten werden.

3. Verfahren gemäß Anspruch 1 oder 2, wobei
die Hauptschnitte (98; 990) entlang des Umfangs des proximalen passiven flexiblen Abschnittes (C) in unterbrochener Weise so geschnitten werden, dass nicht geschnittene Stege (97; 992) zwischen auf einer Umfangslinie liegenden Hauptschnitt-abschnitten (98A, 98B; 990A, 990B, 990C) belassen bleiben.

4. Verfahren gemäß Anspruch 3, wobei
die Nebenschnitte (99; 991) jeweils benachbart zu einem Steg (97; 992) zwischen auf einer Umfangslinie liegenden Hauptschnitt-abschnitten (98A, 98B; 990A, 990B, 990C) geschnitten werden.

5. Verfahren gemäß Anspruch 4, wobei
ein Nebenschnitt (99; 991) in Längsrichtung des proximalen passiven flexiblen Abschnittes (C) benachbart zu dem Steg (97; 992) an einer Seite des Steges (97; 992) jeweils geschnitten wird.

6. Verfahren gemäß Anspruch 4, wobei
zwei Nebenschnitte (99; 991) in Längsrichtung des proximalen passiven flexiblen Abschnittes (C) benachbart zu dem Steg (97; 992) an beiden Seiten des Steges (97; 992) jeweils geschnitten werden.

7. Verfahren gemäß einem der Ansprüche 1 bis 6, wobei
die Hauptschnitte (98; 990) breiter als die Nebenschnitte (99; 991) geschnitten werden.

8. Verfahren gemäß einem der Ansprüche 1 bis 7, wobei
der gesamte Einführschlauch (2) inklusive einem Anschlussbereich (D) des proximalen passiven flexiblen Abschnittes (C) an einem Kontrollkörper (3), dem proximalen passiven flexiblen Abschnitt (C), einem Übergangsbereich (B) zwischen dem proximalen passiven flexiblen Abschnitt (C) und dem Abwinkelungsabschnitt (A), und dem Abwinkelungsabschnitt (A) aus einem einzigen Rohrelement hergestellt wird.

9. Verfahren gemäß einem der Ansprüche 1 bis 8, wobei der gesamte Einführschlauch (2) durch Laser geschnitten wird.

10. Endoskop mit einem Einführschlauch,
wobei der Einführschlauch (2) einen proximalen passiven flexiblen Abschnitt (C) und einen distalen Abwinkelungsabschnitt (A) aufweist,
wobei im proximalen passiven flexiblen Abschnitt (C) Schnitte (98, 99; 990, 991) vorgesehen sind, um ein Biegen des proximalen passiven flexiblen Abschnittes (C) zu ermöglichen,
benachbarte Schnitte (98, 99; 990, 991) im proximalen passiven flexiblen Abschnitt (C) ungleich beabstandet sind;
**dadurch gekennzeichnet, dass**
der proximale passive flexible Abschnitt (C) Hauptschnitte (98; 990) aufweist, die in Längsrichtung des proximalen passiven flexiblen Abschnittes (C) zueinander gleich beabstandet (H) sind, und
der proximale passive flexible Abschnitt (C) benachbart zu den Hauptschnitten (98; 990) Nebenschnitte (99; 991) aufweist, die in Längsrichtung des proximalen passiven flexiblen Abschnittes (C) zu den benachbarten Hauptschnitten (98; 990) an einer Seite der Nebenschnitte (99; 991) näher angeordnet (N) sind als zu den benachbarten Hauptschnitten (98; 990) an der anderen Seite der Nebenschnitte (99; 991).

11. Endoskop gemäß Anspruch 10, wobei
die Hauptschnitte (98; 990) parallel zueinander sind.

12. Endoskop gemäß Anspruch 10 oder 11, wobei
die Hauptschnitte (98; 990) entlang des Umfangs des proximalen passiven flexiblen Abschnittes (C) in unterbrochener Weise so verlaufen, dass nicht geschnittene Stege (97; 992) zwischen auf einer Umfangslinie liegenden Hauptschnitt-abschnitten (98A, 98B; 990A, 990B, 990C) belassen sind.

13. Endoskop gemäß Anspruch 12, wobei
die Nebenschnitte (99; 991) jeweils benachbart zu einem Steg (97; 992) zwischen auf einer Umfangslinie liegenden Hauptschnitt-abschnitten (98A, 98B; 990A, 990B, 990C) angeordnet sind.

14. Endoskop gemäß Anspruch 13, wobei
ein Nebenschnitt (99; 991) in Längsrichtung des proximalen passiven flexiblen Abschnittes (C) benachbart zu dem Steg (97; 992) an einer Seite des Steges (97; 992) jeweils angeordnet ist.

15. Endoskop gemäß Anspruch 13, wobei
zwei Nebenschnitte (99; 991) in Längsrichtung des proximalen passiven flexiblen Abschnittes (C) benachbart zu dem Steg (97; 992) an beiden Seiten des Steges (97; 992) jeweils angeordnet sind.

16. Endoskop gemäß einem der Ansprüche 10 bis 15, wobei
die Hauptschnitte (98; 990) breiter als die Nebenschnitte (99; 991) sind.

17. Endoskop gemäß einem der Ansprüche 10 bis 16, wobei
der gesamte Einführschlauch (2) inklusive einem Anschlussbereich (D) des proximalen passiven flexiblen Abschnittes (C) an einem Kontrollkörper (3), dem proximalen passiven flexiblen Abschnitt (C), einem Übergangsbereich (B) zwischen dem proximalen passiven flexiblen Abschnitt (C) und dem Abwinkelungsabschnitt (A), und dem Abwinkelungsabschnitt (A) aus einem einzigen Rohrelement hergestellt ist.

18. Endoskop gemäß einem der Ansprüche 10 bis 17, wobei
der gesamte Einführschlauch (2) durch Laser geschnitten ist.

## Claims

1. A method for manufacturing an insertion tube (2) of an endoscope from a tubular element,
wherein the insertion tube (2) comprises a proximal passive flexible portion (C) and a distal bending portion (A),
wherein cuts (98, 99; 990, 991) are provided in the proximal passive flexible portion (C), for enabling bending of the proximal passive flexible portion (C,)
wherein the cuts (98, 99; 990, 991) in the proximal passive flexible portion (C) are formed such that adjacent cuts (98, 99; 990, 991) are unequally spaced;
**characterized in that**
main cuts (98; 990) are provided in the proximal passive flexible portion (C), said main cuts (98; 990) being equally spaced apart from each other (H) in the longitudinal direction of the proximal passive flexible portion (C), and
adjacent to the main cuts (98; 990), auxiliary cuts (99; 991) are provided in the proximal passive flexible portion (C), said auxiliary cuts (99; 991) being arranged, in the longitudinal direction of the proximal passive flexible portion (C), closer (N) to the adjacent main cuts (98; 990) on one side of the auxiliary cuts (99; 991) than to the adjacent main cuts (98; 990) on the other side of the auxiliary cuts (99; 991).

2. The method according to claim 1, wherein
the main cuts (98; 990) are cut parallel to each other.

3. The method according to claim 1 or 2, wherein
the main cuts (98; 990) are discontinuously cut along the circumference of the proximal passive flexible portion (C) in such a manner that non-cut bridges (97; 992) remain between main cut portions (98A, 98B; 990A, 990B, 990C) disposed on a circumferential line.

4. The method according to claim 3, wherein
the auxiliary cuts (99; 991) are respectively cut adjacent to a bridge (97; 992) between main cut portions (98A, 98B; 990A, 990B, 990C) disposed on a circumferential line.

5. The method according to claim 4, wherein
one auxiliary cut (99; 991) is respectively cut in the longitudinal direction of the proximal passive flexible portion (C) adjacent to the bridge (97; 992) on one side of the bridge (97; 992).

6. The method according to claim 4, wherein
two auxiliary cuts (99; 991) are respectively cut in the longitudinal direction of the proximal passive flexible portion (C) adjacent to the bridge (97; 992) on both sides of the bridge (97; 992).

7. The method according to any of claims 1 to 6, wherein
the main cuts (98; 990) are cut to be wider than the auxiliary cuts (99; 991).

8. The method according to any of claims 1 to 7, wherein
the entire insertion tube (2), inclusive of a connection region (D) of the proximal passive flexible portion (C) on a control body (3), the proximal passive flexible portion (C), a transition region (B) between the proximal passive flexible portion (C) and the bending portion (A), and the bending portion (A), is manufactured from a single tubular element.

9. The method according to any of claims 1 to 8, wherein
the entire insertion tube (2) is cut by laser.

10. An endoscope having an insertion tube,
wherein the insertion tube (2) comprises a proximal passive flexible portion (C) and a distal bending portion (A),
wherein cuts (98, 99; 990, 991) are provided in the proximal passive flexible portion (C), for enabling bending of the proximal passive flexible portion (C),
wherein adjacent cuts (98, 99; 990, 991) are unequally spaced in the proximal passive flexible portion (C);
**characterized in that**
the proximal passive flexible portion (C) comprises main cuts (98; 990) which are equally spaced apart from each other (H) in the longitudinal direction of the proximal passive flexible portion (C), and
the proximal passive flexible portion (C) comprises auxiliary cuts (99; 991) adjacent to the main cuts (98; 990), said auxiliary cuts (99; 991) being arranged, in the longitudinal direction of the proximal passive flexible portion (C), closer (N) to the adjacent main cuts (98; 990) on one side of the auxiliary cuts (99; 991) than to the adjacent main cuts (98; 990) on the other side of the auxiliary cuts (99; 991).

11. The endoscope according to claim 10, wherein
the main cuts (98; 990) are parallel to each other.

12. The endoscope according to claim 10 or 11, wherein
the main cuts (98; 990) extend discontinuously along the circumference of the proximal passive flexible portion (C) in such a manner that non-cut bridges (97; 992) remain between main cut portions (98A, 98B; 990A, 990B, 990C) disposed on a circumferential line.

13. The endoscope according to claim 12, wherein
the auxiliary cuts (99; 991) are respectively arranged adjacent to a bridge (97; 992) between main cut portions (98A, 98B; 990A, 990B, 990C) disposed on a circumferential line.

14. The endoscope according to claim 13, wherein
one auxiliary cut (99; 991) is respectively arranged in the longitudinal direction of the proximal passive flexible portion (C) adjacent to the bridge (97; 992) on one side of the bridge (97; 992).

15. The endoscope according to claim 13, wherein
two auxiliary cuts (99; 991) are respectively arranged in the longitudinal direction of the proximal passive flexible portion (C) adjacent to the bridge (97; 992) on both sides of the bridge (97; 992).

16. The endoscope according to any of claims 10 to 15, wherein
the main cuts (98; 990) are wider than the auxiliary cuts (99; 991).

17. The endoscope according to any of claims 10 to 16, wherein
the entire insertion tube (2), inclusive of a connection region (D) of the proximal passive flexible portion (C) on a control body (3), the proximal passive flexible portion (C), a transition region (B) between the proximal passive flexible portion (C) and the bending portion (A), and the bending portion (A), is manufactured from a single tubular element.

18. The endoscope according to any of claims 10 to 17, wherein
the entire insertion tube (2) is laser-cut.

## Revendications

1. Procédé de fabrication d'un tuyau d'insertion (2) d'un endoscope à partir d'un élément tubulaire,
le tuyau d'insertion (2) possédant une portion flexible passive (C) proximale et une portion à coudage (A) distale,
des coupes (98, 99 ; 990, 991) étant présentes dans la portion flexible passive (C) proximale afin de rendre possible une flexion de la portion flexible passive (C) proximale,
les coupes (98, 99 ; 990, 991) dans la portion flexible passive (C) proximale étant configurées de telle sorte que les coupes (98, 99 ; 990, 991) voisines sont espacées de manière inégale ; **caractérisé en ce que**
des coupes principales (98 ; 990) sont présentes dans la portion flexible passive (C) proximale, lesquelles sont espacées (H) à l'identique entre elles dans la direction longitudinale de la portion flexible passive (C) proximale, et
des coupes secondaires (99 ; 991) voisines des coupes principales (98 ; 990) sont présente dans la portion flexible passive (C) proximale, lesquelles, dans la direction longitudinale de la portion flexible passive (C) proximale, sont disposées plus proches (N) des coupes principales (98 ; 990) voisines d'un côté des coupes secondaires (99 ; 991) que des coupes principales (98 ; 990) voisines de l'autre côté des coupes secondaires (99 ; 991).

2. Procédé selon la revendication 1,
les coupes principales (98 ; 990) étant coupées parallèlement les unes aux autres.

3. Procédé selon la revendication 1 ou 2,
les coupes principales (98 ; 990) étant découpées de manière interrompue le long du pourtour de la portion flexible passive (C) proximale de telle sorte que des éléments jointifs (97 ; 992) non coupés restent en place entre des portions de coupe principale (98A, 98B ; 990A, 990B, 990C) se trouvant sur une ligne de pourtour.

4. Procédé selon la revendication 3,
les coupes secondaires (91 ; 991) étant respectivement découpées adjacentes à un élément jointif (97 ; 992) entre des portions de coupe principale (98A, 98B ; 990A, 990B, 990C) se trouvant sur une ligne de pourtour.

5. Procédé selon la revendication 4,
les coupes secondaires (91 ; 991) dans la direction longitudinale de la portion flexible passive (C) proximale voisines de l'élément jointif (97 ; 992) étant respectivement découpées sur un côté de l'élément jointif (97 ; 992).

6. Procédé selon la revendication 4,
deux coupes secondaires (91 ; 991) dans la direction longitudinale de la portion flexible passive (C) proximale voisines de l'élément jointif (97 ; 992) étant respectivement découpées des deux côtés de l'élément jointif (97 ; 992).

7. Procédé selon l'une des revendications 1 à 6,
les coupes principales (98 ; 990) étant découpées plus larges que les coupes secondaires (91 ; 991).

8. Procédé selon l'une des revendications 1 à 7,
l'ensemble du tuyau d'insertion (2), y compris une zone de raccordement (D) de la portion flexible passive (C) proximale à un corps de contrôle (3), la portion flexible passive (C) proximale, une zone de transition (B) entre la portion flexible passive (C) proximale et la portion à coudage (A), et la portion à coudage (A) étant produit à partir d'un unique élément tubulaire.

9. Procédé selon l'une des revendications 1 à 8,
l'ensemble du tuyau d'insertion (2) étant découpé au laser.

10. Endoscope comprenant un tuyau d'insertion,
le tuyau d'insertion (2) possédant une portion flexible passive (C) proximale et une portion à coudage (A) distale,
des coupes (98, 99 ; 990, 991) étant présentes dans la portion flexible passive (C) proximale afin de rendre possible une flexion de la portion flexible passive (C) proximale,
les coupes (98, 99 ; 990, 991) voisines dans la portion flexible passive (C) proximale étant espacées de manière inégale ;
**caractérisé en ce que**
la portion flexible passive (C) proximale possède des coupes principales (98 ; 990), qui sont espacées (H) à l'identique entre elles dans la direction longitudinale de la portion flexible passive (C) proximale, et
la portion flexible passive (C) proximale possède des coupes secondaires (99 ; 991) voisines des coupes principales (98 ; 990), lesquelles, dans la direction longitudinale de la portion flexible passive (C) proximale, sont disposées plus proches (N) des coupes principales (98 ; 990) voisines d'un côté des coupes secondaires (99 ; 991) que des coupes principales (98 ; 990) voisines de l'autre côté des coupes secondaires (99 ; 991).

11. Endoscope selon la revendication 10,
les coupes principales (98 ; 990) étant parallèles les unes aux autres.

12. Endoscope selon la revendication 10 ou 11,
les coupes principales (98 ; 990) suivant un tracé interrompu le long du pourtour de la portion flexible passive (C) proximale de telle sorte que des éléments jointifs (97 ; 992) non découpés sont laissés entre des portions de coupe principale (98A, 98B ; 990A, 990B, 990C) se trouvant sur une ligne de pourtour.

13. Endoscope selon la revendication 12,
les coupes secondaires (99 ; 991) étant respectivement disposées voisines d'un élément jointif (97 ; 992) entre des portions de coupe principale (98A, 98B ; 990A, 990B, 990C) se trouvant sur une ligne de pourtour.

14. Endoscope selon la revendication 13,
une coupe secondaire (99 ; 991) dans la direction longitudinale de la portion flexible passive (C) proximale voisine de l'élément jointif (97 ; 992) étant respectivement disposée d'un côté de l'élément jointif (97 ; 992).

15. Endoscope selon la revendication 13,
deux coupes secondaires (99 ; 991) dans la direction longitudinale de la portion flexible passive (C) proximale voisines de l'élément jointif (97 ; 992) étant respectivement disposées des deux côtés de l'élément jointif (97 ; 992).

16. Endoscope selon l'une des revendications 10 à 15,
les coupes principales (98 ; 990) étant plus larges que les coupes secondaires (99 ; 991).

17. Endoscope selon l'une des revendications 10 à 16,
l'ensemble du tuyau d'insertion (2), y compris une zone de raccordement (D) de la portion flexible passive (C) proximale à un corps de contrôle (3), la portion flexible passive (C) proximale, une zone de transition (B) entre la portion flexible passive (C) proximale et la portion à coudage (A), et la portion à coudage (A) étant produit à partir d'un unique élément tubulaire.

18. Endoscope selon l'une des revendications 10 à 17,
l'ensemble du tuyau d'insertion (2) étant découpé au laser.
